(19) Europäisches Patentamt
European Patent Office
Office européen des brevets

(11) Veröffentlichungsnummer: **0 065 743**
**B1**

(12) # EUROPÄISCHE PATENTSCHRIFT

(45) Veröffentlichungstag der Patentschrift: **31.07.85**

(51) Int. Cl.[4]: **C 09 B 57/08,** C 09 B 57/02, C 07 D 493/06, C 07 D 491/06

(21) Anmeldenummer: **82104346.0**

(22) Anmeldetag: **18.05.82**

(54) **Neue Cumarinverbindungen, Verfahren zu ihrer Herstellung und ihre Verwendung als Farbstoffe.**

(30) Priorität: **22.05.81 DE 3120402**

(43) Veröffentlichungstag der Anmeldung: **01.12.82 Patentblatt 82/48**

(45) Bekanntmachung des Hinweises auf die Patenterteilung: **31.07.85 Patentblatt 85/31**

(84) Benannte Vertragsstaaten: **BE CH DE FR GB IT LI NL**

(56) Entgegenhaltungen:
**DE - A - 2 436 032**
**DE - B - 1 098 125**
**FR - A - 2 353 615**
**GB - A - 2 006 253**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Deger, Hans-Matthias, Dr., Am Rheingauer Weg 6, D-6238 Hofheim am Taunus (DE)**
Erfinder: **Erckel, Rüdiger, Dr., Staufenstrasse 16, D-6239 Eppstein/Taunus (DE)**
Erfinder: **Frühbeis, Horst, Dr., Am Mannstein 8, D-6233 Kelkheim (Taunus) (DE)**

**EP 0 065 743 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Gegenstand der Erfindung sind neue Cumarinverbindungen der allgemeinen Formel (1)

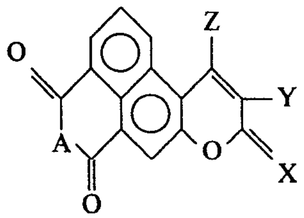

(1)

in welcher A ein Sauerstoffatom oder die Gruppierung $>N-R$ bedeutet, worin R ein Wasserstoffatom oder einen gegebenenfalls durch Fluor-, Chlor- oder Bromatome oder einen gegebenenfalls beispielsweise durch Alkoxy$_{C_1-C_4}$, Dialkyl$_{C_1-C_4}$amino-, $-SO_2-N(alkyl_{C_1-C_4})_2$- oder Cyangruppen substituierten Alkylrest von 1 bis 18 Kohlenstoffatomen, oder einen gegebenenfalls beispielsweise durch Fluor-, Chlor- oder Bromatome oder Alkyl$_{C_1-C_4}$, Alkoxy$_{C_1-C_4}$, Hydroxyl-, Cyan-, Sulfonamid- oder $-SO_2-N(alkyl_{C_1-C_4})_2$-Gruppen substituierten cycloaliphatischen, aromatischen oder heterocyclischen Rest darstellt, wobei als cycloaliphatischer Rest beispielsweise ein Cyclopentyl-, Cyclohexyl- oder Cycloheptylrest, als aromatischer Rest beispielsweise ein Phenyl- oder Naphthylrest, und als heterocyclischer Rest beispielsweise ein Pyridin-, Pyrimidin-, Benzoxazolyl-2-, Benzthiazolyl-2, Benzimidazolyl-2-Rest in Frage kommt, X ein Sauerstoffatom oder die Gruppierung N—R' bedeutet, worin R' ein Wasserstoffatom oder eine Alkyl$_{C_1-C_{18}}$carbonyl-, Alkoxy$_{C_1-C_4}$carbonyl-, Alkyl$_{C_1-C_4}$carbamoyl-, Alkyl$_{C_1-C_4}$sulfonylgruppe, oder eine gegebenenfalls beispielsweise durch Fluor-, Chlor- oder Bromatome oder Alkyl- oder Alkoxygruppen von jeweils 1 bis 4 Kohlenstoffatomen oder Cyangruppen substituierte Arylgruppe (wie beispielsweise die Phenylgruppe), Arylcarbonylgruppen (wie beispielsweise die Phenylcarbonylgruppe), Arylcarbamoylgruppe (wie beispielsweise die Phenylcarbamoylgruppe) oder Arylsulfonylgruppe (wie beispielsweise die Phenylsulfonylgruppe) darstellt, Y ein Wasserstoffatom oder eine Cyan- oder Carbonsäurealkyl$_{C_1-C_4}$estergruppe, eine gegebenenfalls beispielsweise durch Fluor-, Chlor- oder Bromatome oder Alkyl- oder Alkoxygruppen von 1 bis 4 Kohlenstoffatomen, Nitro-, Methylendioxy-, Amino-, Monoalkyl$_{C_1-C_4}$amino-, Dialkyl$_{C_1-C_4}$aminogruppen oder einen Benzoxazolylrest substituierten Arylrest (wie beispielsweise den Phenylrest), Arylsulfonylrest (wie beispielsweise den Phenylsulfonylrest) oder —CO—O—aryl-Rest (wie beispielsweise den —CO—O-phenyl-Rest), einen gegebenenfalls quaternierten und gegebenenfalls beispielsweise durch Fluor-, Chlor- oder Bromatome oder Alkyl- oder Alkoxygruppen von 1 bis 4 Kohlenstoffatomen, Nitro-, Phenyl-, Alkyl$_{C_1-C_4}$phenyl-, Alkoxy$_{C_1-C_4}$phenyl-, Cyan-, Methylendioxy- oder Carbonsäurealkyl$_{C_1-C_4}$estergruppen substituierten heterocyclischen Rest, beispielsweise einen Thiophen-, Benzothiophen-, Furan-, Benzofuran-, Triazol-, Imidazol-, Benzimidazol-, Thiazol-, Benzthiazol-, Oxazol-, Benzoxazol-, Thiadiazol-, Oxadiazol- oder Pyridinrest, und Z ein Wasserstoffatom oder eine Carbonsäurealkyl$_{C_1-C_4}$ester- oder Cyangruppe bedeutet, sowie ein Verfahren zu ihrer Herstellung und ihre Verwendung als Farbstoffe zum Färben oder Bedrucken von synthetischen Fasermaterialien, die gegebenenfalls in Mischung mit natürlichen Fasermaterialien vorliegen.

Die Farbstoffe der weiter oben genannten allgemeinen Formel (1) können hergestellt werden, indem man eine Verbindung der allgemeinen Formel (2)

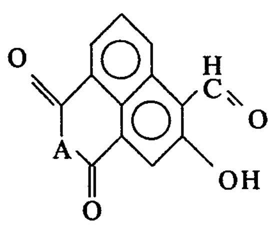

(2)

in welcher A die vorstehend angegebenen Bedeutungen hat

a) mit einer Verbindung der allgemeinen Formel (3)

$$NC-CH_2-Y \qquad (3)$$

in welcher Y die vorstehend genannten Bedeutungen hat, in an sich bekannter Weise kondensiert (DE-OS 1 619 567) und die hierbei erhaltene Iminocumarinverbindung der allgemeinen Formel (4)

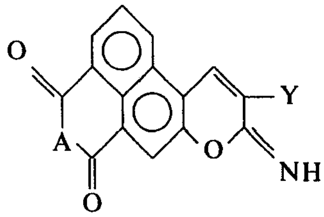

(4)

in welcher A und Y die weiter oben genannten Bedeutungen haben, gegebenenfalls nach an sich bekannten Verfahren (DE-OS 2 234 207) acyliert, mit Arylaminen behandelt oder verseift, oder

b) mit einer Verbindung der allgemeinen Formel (5)

$$R''O-\underset{\underset{O}{\|}}{C}-CH_2-Y \qquad (5)$$

in welcher Y die weiter oben genannte Bedeutung hat und R″ einen gegebenenfalls substituierten Alkyl- oder Arylrest bedeutet, in an sich bekannter Weise kondensiert und die hierbei erhaltene Cumarinverbindung der allgemeinen Formel (6)

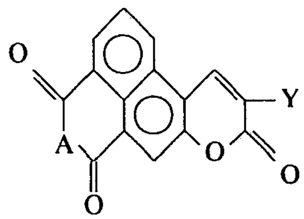

(6)

in welcher A und Y die weiter obengenannten Bedeutungen haben, gegebenenfalls nach an sich bekannten Verfahren mit einem Cyanidsalz umsetzt und die als Zwischenprodukt gebildete Anlagerungsverbindung gleichzeitig oder nachträglich mit einem Oxidationsmittel behandelt unter Erhalt der Verbindung der allgemeinen Formel (7)

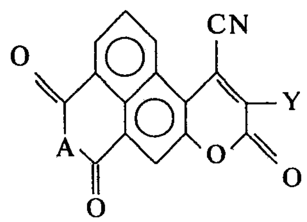

(7)

Die Verbindungen (Aldehyde) der weiter oben genannten allgemeinen Formel (2) können auf die in der DE-OS 24 36 032 beschriebene Weise hergestellt werden, indem man 3-Hydroxynaphthalsäureanhydrid bzw. 3-Hydroxynaphthalimid in Gegenwart einer niederen aliphatischen Carbonsäure, beispielsweise Ameisensäure oder Essigsäure, mit Hexamethylentetramin oder einem Gemisch aus Ammoniak und Formaldehyd in Gegenwart einer geringen Menge Mineralsäure, wie Schwefelsäure oder Salzsäure, bei Temperaturen zwischen vorzugsweise 80 und 100°C erhitzt.

Die Kondensation der Aldehyde der genannten allgemeinen Formel (2) mit den Verbindungen der genannten allgemeinen Formel (3) erfolgt in einem polaren organischen Lösemittel in Gegenwart anorganischer oder organischer Kondensationsmittel und bei Temperaturen zwischen 0° und 100°C, vorzugsweise 20° und 80°C. Als polare organische Lösungsmittel eignen sich beispielsweise Alkohole, insbesondere niedrige Alkanole, wie Methanol oder Äthanol, ferner cyclische Äther, wie Dioxan oder Tetrahydrofuran, stickstoffhaltige aromatische Lösemittel, wie z. B. Pyridin oder Picolin und vor allem Amide niederer Fettsäuren, wie Formamid oder Dimethylformamid. Als basische Kondensationsmittel eignen sich beispielsweise die Alkalisalze der Kohlensäure, wie Natrium- oder Kaliumcarbonat, Alkalimetallsalze niederer Fettsäuren, wie Natrium- oder Kaliumacetat, oder Alkalimetallalkoholate, wie Natrium- oder Kalium-methanolat oder -äthanolat, oder Stickstoffbasen, wie Piperidin oder Pyrrolidin.

Die Acylierung der Iminocumarine der genannten allgemeinen Formel (4) erfolgt mit Carbonsäurehalogeniden, Chlorameisensäureestern, Carbonsäureanhydriden oder Sulfonsäurehalogeniden in einem Lösungsmittel in Gegenwart einer anorganischen oder organischen Base, vorzugsweise eines Amins, bei Temperaturen zwischen 0° und 160°C, vorzugsweise 20° und 100°C. Als Lösemittel eignen sich besonders solche organischer Natur, wie beispielsweise Kohlenwasserstoffe wie Benzol, Toluol oder Xylol, ferner halogenierte Kohlenwasserstoffe, wie Chloroform oder Chlorbenzol, Äther, wie beispielsweise Diäthyläther, Glykoldimethyläther, Dioxan und Tetrahydrofuran, oder Aceton.

Geeignete Basen sind hierbei beispielsweise Alkalicarbonate, wie Natrium- oder Kaliumcarbonat,

Alkalisalze niederer Fettsäuren, wie Natrium- oder Kaliumacetat, und insbesondere organische Basen, wie beispielsweise Triäthylamin, Pyridin, Pyrrolidin oder N,N-Dimethylanilin. Die organischen Basen können hierbei auch als Lösemittel dienen.

Als geeignete Carbonsäureanhydride seien beispielsweise Acetanhydrid oder Propionsäureanhydrid genannt, als Säurehalogenide beispielsweise Acetylchlorid, Chloracetylchlorid, Stearylchlorid, Benzoylchlorid, 3-Nitrobenzoylchlorid, 3,5-Dinitrobenzoylchlorid, 4-Chlorbenzoylchlorid, Zimtsäurechlorid, Methansulfonsäurechlorid, Äthansulfonsäurechlorid, Benzolsulfonsäurechlorid, Toluolsulfonsäurechlorid, 3-Methoxypropansulfochlorid, Cyanmethansulfochlorid, Chloräthansulfochlorid, Chlorameisensäuremethylester, Chlorameisensäurephenylester, Chlorameisensäure-$\beta$-methoxyäthylester, Chlorameisensäurediäthylamid.

Die Umsetzung (Acylierung) der Iminocumarine der weiter oben genannten allgemeinen Formel (4) mit Isocyanaten, gegebenenfalls in einem inerten, wasserfreien Lösungsmittel, wie beispielsweise Toluol, Chloroform, Chlorbenzol oder Dioxan, wird bei Temperaturen zwischen 0° und 150°C, vorzugsweise bei 50° bis 100°, vorgenommen.

Die Umsetzung der Iminocumarine der genannten allgemeinen Formel (4) mit Arylaminen, beispielsweise Anilinen, die im aromatischen Kern durch Fluor-, Chlor- oder Bromatome oder Alkyl- oder Alkoxygruppen von jeweils 1 bis 4 Kohlenstoffatome oder Cyangruppen substituiert sein können, erfolgt nach Methoden, wie sie beispielsweise in der DE-OS 2 226 211 beschrieben sind. Man erhitzt hierbei die Iminocumarine beispielsweise mit einem Anilinderivat auf Temperaturen von 50–200°C, vorzugsweise auf 80–150°C, wobei das Anilinderivat selbst als Lösemittel dienen kann. Weitere geeignete Lösemittel sind hierbei beispielsweise Alkohole, wie Propanol, Butanol, Glykole sowie deren Mono- und Diäther, vorzugsweise Glykolmonoäthyläther, sowie Dimethylformamid, N-Methylpyrrolidon oder Dimethylsulfoxid. Kohlenwasserstoffe, wie Toluol und Xylol, sowie Chlorkohlenwasserstoffe, wie Chlorbenzol, Dichlorbenzol oder Perchloräthylen kommen ebenfalls in Betracht.

Die Verbindungen der genannten Formel (1) mit X = Sauerstoff und Z = Wasserstoff können hergestellt werden, indem man die Verbindungen (Aldehyde) der weiter oben genannten allgemeinen Formel (2) mit Verbindungen der allgemeinen Formel (5)

$$R''O-\underset{\underset{O}{\|}}{C}-CH_2-Y \qquad (5)$$

in welcher R'' und Y die weiter oben genannten Bedeutungen haben, unter den Bedingungen der Knoevenagel-Kondensation umsetzt, oder indem man die Iminocumarine der weiter oben genannten allgemeinen Formel (4) nach an sich bekannten Verfahren verseift. Die Verseifung der Iminocumarinverbindungen der allgemeinen Formel (4) zu den Cumarinen der allgemeinen Formel (1) mit X = Sauerstoff und Z = Wasserstoff erfolgt mit verdünnten wäßrigen Säuren bei Temperaturen zwischen 80 und 120°C. Als Säuren eignen sich hierbei Mineralsäuren, wie Salzsäure, Bromwasserstoffsäure oder Schwefelsäure, aber auch organische Säuren, wie Ameisensäure, Essigsäure oder Toluolsulfonsäure, in Konzentrationen von 20–200 g/l, vorzugsweise von 40–120 g/l.

Die Kondensation der weiter oben genannten Verbindungen (Aldehyde) der Formel (2) mit den Verbindungen der allgemeinen Formel (5) erfolgt in einem organischen Lösemittel in Gegenwart anorganischer oder organischer Kondensationsmittel und bei Temperaturen zwischen 50 und 200°C, vorzugsweise 80 bis 150°C, unter gleichzeitiger Entfernung des Reaktionswassers. Als Lösemittel eignen sich Kohlenwasserstoffe, wie Toluol, Xylol oder Cyclohexan, chlorierte Kohlenwasserstoffe, wie Chlorbenzol, Chloroform oder Trichloräthylen, Alkohole wie Methanol, Äthanol oder Propanol sowie stickstoffhaltige Lösemittel, wie Pyridin, Picolin, Dimethylformamid und N,N-Dimethylanilin. Als Kondensationsmittel eignen sich Alkalimetallhydroxide, wie Natrium- und Kaliumhydroxid, Alkalimetallalkoholate, wie Natrium- und Kaliummethanolat oder Natrium- und Kaliumäthanolat, Stickstoffbasen, wie Piperidin, Pyrrolidin und N-Methylanilin, sowie Salze von Stickstoffbasen, wie Piperazin und Ammoniumacetat.

Die Verbindungen der weiter oben genannten allgemeinen Formel (7) können hergestellt werden, indem man die Verbindungen der genannten allgemeinen Formel (6) nach an sich bekannten Verfahren, wie sie beispielsweise in der DE-OS 28 44 299 niedergelegt sind, in einem Lösemittel mit einem Cyanidsalz umsetzt, und die hierbei als Zwischenprodukte gebildeten Anlagerungsverbindungen gleichzeitig oder nachträglich mit Oxidationsmitteln behandelt.

Als Cyanidsalze verwendet man hierbei vorzugsweise wasserlösliche Alkalicyanide, wahlweise als Feststoff oder insbesondere in Form von konzentrierten wäßrigen Lösungen.

Geeignete Lösemittel sind polare organische Lösemittel wie Alkohole, z. B. Methanol, Äthanol, Isopropanol und Äthylenglykol, insbesondere aber aprotische dipolare Lösemittel, wie Dimethylformamid, Pyridin und Picolin.

Geeignete Oxidationsmittel sind Wasserstoffperoxid, Persulfate und organische Peroxide, wie z. B. Dibenzoylperoxid, Salpetersäure, Brom und Bleitetracetat.

Die Umsetzung der Cyanidsalze wird im Temperaturbereich von 0–100°C, vorzugsweise 15–70°C und die Oxidation im Bereich von –10 bis 30°C, vorzugsweise bei –5 bis 10°C durchgeführt.

Die neuen Cumarinverbindungen der genannten Formel (1) stellen gelbe bis schwarz-rote kristalline Pulver dar, die sich in organischen Lösemitteln, wie Alkoholen, Estern, Amiden, Äthern, Ketonen und chlorierten Kohlenwasserstoffen, mit intensiver grüngelber bis roter Fluoreszenz lösen.

Die neuen Verbindungen eignen sich als Farbstoffe allein oder in Mischungen untereinander oder mit anderen Farbstoffen, vorzugsweise in zubereiteter Form, wie beispielsweise in wäßriger Dispersion oder in Lösung in organischen Lösemitteln oder in Emulsionen oder Dispersionen, die neben einem Lösemittel oder einem Lösemittelgemisch noch Wasser enthalten können, zum Färben oder Bedrucken von synthetischen Fasermaterialien, wie beispielsweise solchen aus Cellulose-di-, $2^1/_2$- und -triacetat, Polyamiden, wie Poly-$\varepsilon$-caprolactam oder Poly-hexamethylen-diaminadipat, ferner solchen aus Polyurethanen oder Polycarbonaten, insbesondere aber solchen aus linearen Polyestern, wie Polyäthylenterephthalaten.

Die vorstehend genannten synthetischen Fasermaterialien können zum Färben oder Bedrucken auch in Mischungen untereinander oder mit natürlichen Fasermaterialien, wie Cellulosefasern oder Wolle, vorliegen. Ferner können sie zum Färben in verschiedenen Verarbeitungszuständen, beispielsweise als Kammzug, Flocke, Fäden, Gewebe oder Gewirke, vorliegen.

Die Applikation der erfindungsgemäßen Farbstoffe erfolgt in prinzipiell bekannter Weise, in der Regel aus wäßriger Dispersion, kann jedoch auch aus organischen Lösungsmitteln vorgenommen werden. Die Dispergierung der Farbstoffe kann beispielsweise durch Mahlen in Gegenwart eines Dispergiermittels, wie beispielsweise des Kondensationsproduktes aus Formaldehyd und technischem m-Kresol und Natriumbisulfit, das mit Schwefelsäure neutral gestellt wurde, vorgenommen werden. Im übrigen richten sich die Färbebedingungen weitgehend nach der Art der vorliegenden synthetischen Fasermaterialien und deren Verarbeitungszustand.

Beispielsweise erfolgt das Färben von geformten Gebilden aus Celluloseacetat in einem Temperaturbereich von 75 bis 85°C. Cellulosetriacetatfasern werden bei Temperaturen zwischen etwa 90 und 125°C gefärbt. Das Aufbringen der Farbstoffe auf Polyamidfasermaterialien geschieht im Temperaturbereich zwischen etwa 90 und 120°C. Für das Färben von Fasermaterialien auf Polyestern benutzt man die hierfür bekannten Methoden, indem man das Fasermaterial in Gegenwart von Carriern, wie o- oder p-Phenylphenol, Methylnaphthalin oder Methylsalicylat, bei Temperaturen von 100°C bis etwa 130°C oder aber ohne Anwendung von Carriern bei entsprechenden höheren Temperaturen, beispielsweise zwischen 120 und 140°C färbt. Außerdem kann man auch so verfahren, daß man die Farbstoffe durch Klotzen mit oder ohne Verdickungsmittel, z. B. Tragant-Verdickung, aufbringt und durch Hitzeeinwirkung, beispielsweise durch Dampf oder Trockenhitze während $^1/_2$ bis 30 Minuten bei Temperaturen im Bereich von etwa 100 bis 230°C fixiert. Das so gefärbte Material wird dann zur Verbesserung der Reibechtheit zweckmäßig von oberflächlich anhaftendem Farbstoff befreit, beispielsweise durch Spülen oder eine reduktive Nachbehandlung. Diese Nachbehandlung erfolgt im allgemeinen bei 60 bis 120°C in einer wäßrigen Natronlauge, Natriumdithionit und ein nichtionogenes Waschmittel, wie beispielsweise ein Äthylenoxyd-Phenol-Additionsprodukt, enthaltenden Flotte.

Zur Färbung der synthetischen Fasermaterialien aus organischen Lösemitteln kann man beispielsweise so verfahren, daß man bei Raumtemperatur oder darüber, vorzugsweise bei 70 bis 130°C, gegebenenfalls unter Druck, den Farbstoff aus der Lösung auf die Faser aufziehen läßt oder so, daß man in einer kontinuierlichen Arbeitsweise Gewebe oder Gewirke mit einer Farbstofflösung imprägniert, trocknet und einer kurzzeitigen Hitzeeinwirkung, beispielsweise bei Temperaturen von 180 bis 210°C, unterwirft. Als Lösemittel für das Ausziehverfahren seien beispielsweise mit Wasser nicht mischbare Lösemittel mit Siedepunkten zwischen 40 und 170°C genannt, wie etwa die aliphatischen Halogenkohlenwasserstoffe, wie Methylenchlorid, Trichloräthan, Trichloräthylen oder Trifluortrichloräthan. Insbesondere für ein kontinuierliches Färbeverfahren kommen auch mit Wasser mischbare Lösemittel, wie beispielsweise Alkohole oder Dimethylformamid, in Betracht. Die Lösemittel können natürlich auch als Mischungen vorliegen und weitere in Lösemitteln lösliche Hilfsmittel, wie beispielsweise Oxalkylierungsprodukte von Fettalkoholen, Alkylphenolen und Fettsäuren, enthalten.

Zur Herstellung von Drucken auf den synthetischen Fasermaterialien, beispielsweise solchen aus Polyestern, Polyamiden oder Cellulosetriacetat, können die Farbstoffe in Form wasserhaltiger Zubereitungen angewandt werden, die neben dem fein verteilten Farbstoff geeignete Verdickungsmittel enthalten können. Die Fixierung erfolgt beispielsweise nach dem Drucken und Trocknen durch Dämpfen bei Atmosphärendruck oder unter erhöhtem Druck bis zu 2,5 atü während 10 bis 60 Minuten. Ebenso kann man die Fixierung durch die Einwirkung von Heißluft von 160 bis 120°C während 30 Sekunden bis 10 Minuten bewirken.

Zusätzlich eignen sich die erfindungsgemäßen Farbstoffe zum Schmelzspinnfärben, vorzugsweise von Polyäthylenterephthalat.

Mit den erfindungsgemäßen neuen Farbstoffen lassen sich die genannten Fasern, Gewebe und Kunststoffmassen zum Teil extrem brillant grünstichig-gelb bis rot fluoresazierend färben. Die Färbungen zeichnen sich im Vergleich zu den bislang bekannten 5,6-Benzocumarinen aus der DE-A-2 8 44 299 durch hohe Farbstärke, gutes Aufbau- und Ziehvermögen, sowie durch sehr gute Echtheitseigenschaften, wie Wasch-, Reib-, Sublimier-, Schweiß- und Lichtechtheiten aus. Die in der FR-A 2 353 615 beschriebenen Farbstoffe aus der Benzobenzimidazo(1,2-a)-chinolin-Reihe weisen demgegenüber nur unzureichende Eignung zur Färbung für Polyesterfasermaterial auf; sie sind hingegen mehr für den Zweck der Pigmentierung von Lacken geeignet.
Die in den nachstehenden Beispielen angegebenen Teile bedeuten Gewichtsteile.

Beispiel 1

255 Teile 3-Oxi-4-formyl-N-methylnaphthalimid und 157 Teile Benzimidazolyl-2-acetonitril werden in 5000 Teilen Dimethylformamid suspendiert und unter Rühren mit 20 Teilen Piperidin versetzt. Nach vierstündigem Rühren bei Raumtemperatur wird das ausgefallene Produkt abgesaugt, mit kaltem Äthanol gewaschen und bei 50°C im Vakuum getrocknet. Gewonnen werden 362 Teile (92% der Theorie) orange gefärbtes Farbstoffpulver der Formel

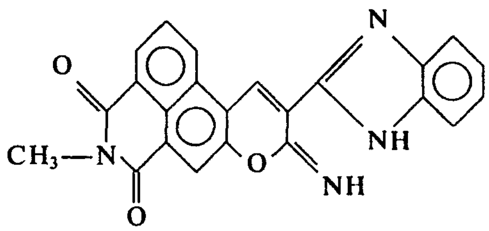

das auf Polyesterfasermaterialien brillante gelb-rote Färbungen ergibt.

Beispiel 2

313 Teile 3-Oxi-4-formyl-N-(3-methoxy-n-propyl)-naphthalimid und 157 Teile Benzimidazolyl-2-acetonitril werden in 3000 Teilen Dimethylformamid suspendiert und unter Rühren mit 10 Teilen Piperidin versetzt. Nach zweistündigem Rühren bei Raumtemperatur wird das ausgefallene Produkt abgesaugt, mit kaltem Äthanol gewaschen und bei 50°C im Vakuum getrocknet. Gewonnen werden 393 Teile (87% der Theorie) gelbes Farbstoffpulver der Formel

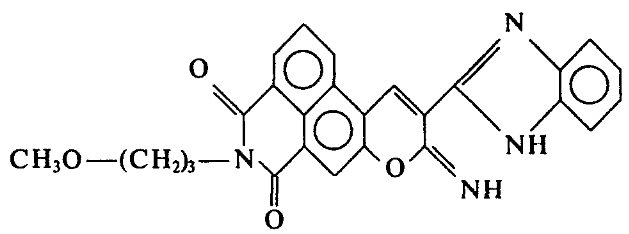

das auf Polyesterfasermaterialien brillante gelb-rote Färbungen ergibt.

Beispiel 3

323 Teile 3-Oxi-4-formyl-N-cyclohexylnaphthalimid und 157 Teile Benzimidazolyl-2-acetonitril werden in 3500 Teilen Dimethylformamid suspendiert und unter Rühren mit 15 Teilen Piperidin versetzt. Nach siebenstündigem Rühren bei Raumtemperatur wird das ausgefallene Produkt abgesaugt, mit kaltem Äthanol gewaschen und bei 50°C im Vakuum getrocknet. Gewonnen werden 394 Teile (85% der Theorie) gelbes Farbstoffpulver der Formel

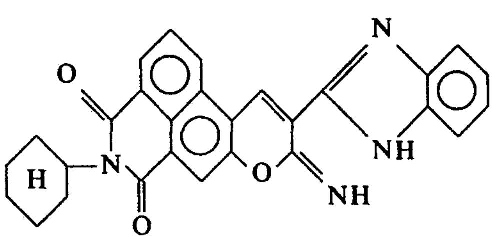

das auf Polyesterfasermaterialien brillante gelb-rote Färbungen ergibt.

Beispiel 4

313 Teile 3-Oxi-4-formyl-N-(3-methoxy-n-propyl)-naphthalimid und 187 Teile 5-Methoxy-2-cyanmethyl-benzimidazol werden in 5000 Teilen Dimethylformamid suspendiert und unter Rühren mit 15 Teilen Pyrrolidin versetzt. Nach fünfstündigem Rühren bei Raumtemperatur wird das ausgefallene Produkt abgesaugt, mit kaltem Äthanol gewaschen und bei 50°C im Vakuum getrocknet. Gewonnen werden 463 Teile (96% der Theorie) rotes Farbstoffpulver der Formel

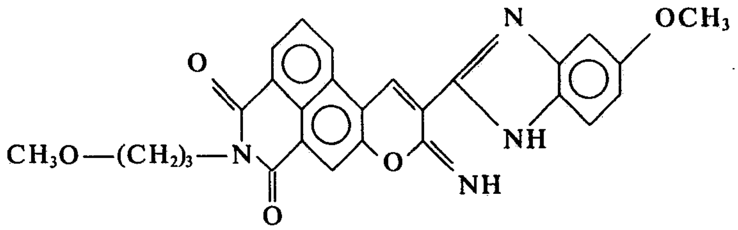

das auf Polyesterfasermaterialien brillante orange Färbungen ergibt.

Beispiel 5

313 Teile 3-Oxi-4-formyl-N-(3-methoxy-n-propyl)-naphthalimid und 217 Teile 5,6-Dimethoxy-2-cyanmethyl-benzimidazol werden in 2500 Teilen Dimethylformamid suspendiert und unter Rühren mit 10 Teilen Piperidin versetzt. Nach dreistündigem Rühren bei Raumtemperatur wird das ausgefallene Produkt abgesaugt, mit kaltem Äthanol gewaschen und bei 50°C im Vakuum getrocknet. Gewonnen werden 460 Teile (90% der Theorie) dunkelrotes Farbstoffpulver der Formel

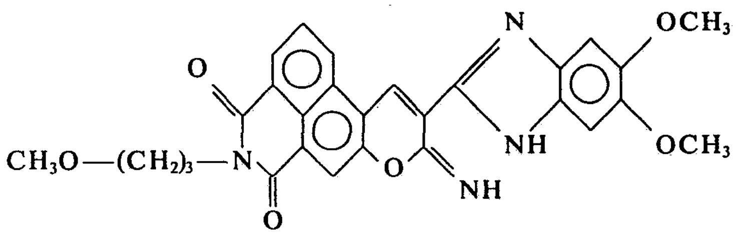

das auf Polyesterfasermaterialien rote Färbungen ergibt.

Verfährt man analog den Beispielen 1–5, so erhält man weitere erfindungsgemäße Farbstoffe, die in der nachstehenden Tabelle aufgeführt sind:

Allgemeine Formel der Farbstoffe der nachstehenden Tabellenbeispiele 6–67:

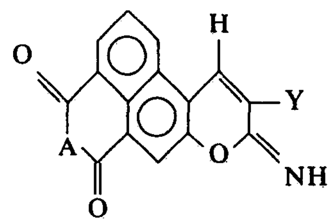

| Beispiel Nr. | A | Y | Farbton auf Polyesterfasern |
|---|---|---|---|
| 6 | $N-CH_3$ | 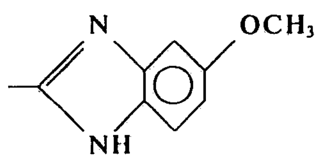 | brillantes Orange |
| 7 | 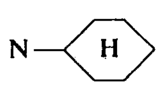 | desgl. | desgl. |
| 8 | $N-CH_3$ | 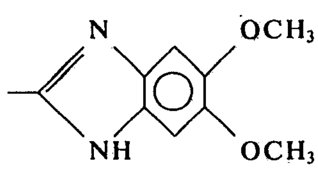 | Rot |
| 9 | 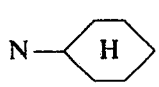 | desgl. | desgl. |

Fortsetzung

| Beispiel Nr. | A | Y | Farbton auf Polyesterfasern |
|---|---|---|---|
| 10 | O | 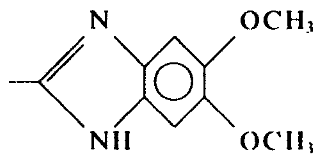 | Rot |
| 11 | $N-CH_3$ | 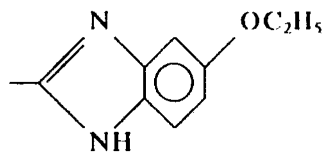 | brillantes Orange |
| 12 | $N-(CH_2)_3-OCH_3$ | desgl. | desgl. |
| 13 | O | desgl. | desgl. |
| 14 | 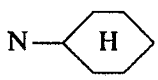 | desgl. | desgl. |
| 15 | $N-CH_3$ | 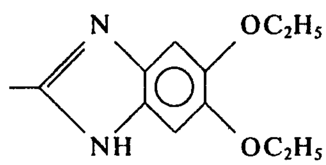 | Rot |
| 16 | $N-(CH_2)_3-OCH_3$ | desgl. | desgl. |
| 17 | $N-CH_3$ | 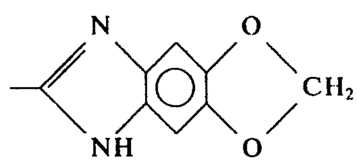 | desgl. |
| 18 | $N-(CH_2)_3-OCH_3$ | desgl. | desgl. |
| 19 | O | 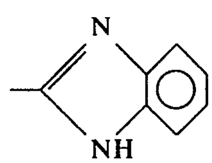 | brillantes Gelb |
| 20 | 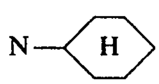 | desgl. | desgl. |
| 21 | $N-CH_3$ | 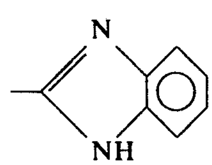 | brillantes Grüngelb |
| 22 | $N-(CH_2)_3-OCH_3$ | desgl. | desgl. |
| 23 | O | desgl. | desgl. |
| 24 | 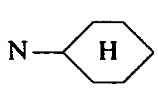 | desgl. | desgl. |

Fortsetzung

| Beispiel Nr. | A | Y | Farbton auf Polyesterfasern |
|---|---|---|---|
| 25 | N—$CH_3$ | 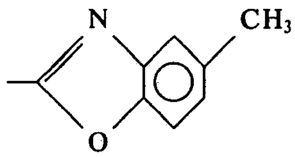 | brillantes Grüngelb |
| 26 | N—$(CH_2)_3$—$OCH_3$ | desgl. | desgl. |
| 27 | N—$CH_3$ | 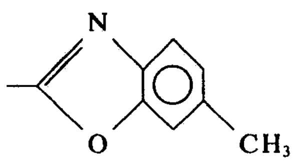 | brillantes Gelb |
| 28 | N—$(CH_2)_3$—$OCH_3$ | desgl. | desgl. |
| 29 | N—$CH_3$ | desgl. | desgl. |
| 30 | N—$(CH_2)_3$—$OCH_3$ | desgl. | desgl. |
| 31 | N—$CH_3$ | 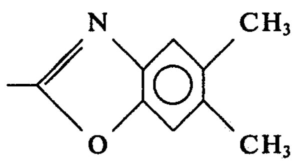 | desgl. |
| 32 | N—$(CH_2)_3$—$OCH_3$ | desgl. | desgl. |
| 33 | N—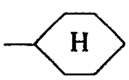 | desgl. | desgl. |
| 34 | N—$CH_3$ | 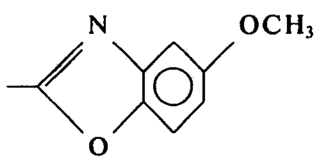 | desgl. |
| 35 | N—$(CH_2)_3$—$OCH_3$ | 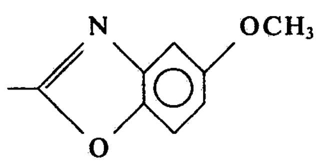 | desgl. |
| 36 | N—$CH_3$ | 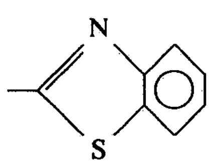 | desgl. |
| 37 | N—$(CH_2)_3$—$OCH_3$ | desgl. | desgl. |
| 38 | N—$CH_3$ | 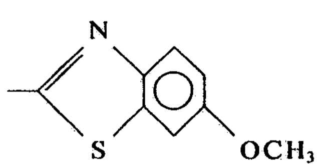 | desgl. |

Fortsetzung

| Beispiel Nr. | A | Y | Farbton auf Polyesterfasern |
|---|---|---|---|
| 39 | $N-(CH_2)_3-OCH_3$ | 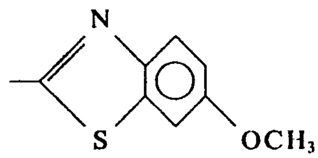 | brillantes Gelb |
| 40 | $N-CH_3$ | 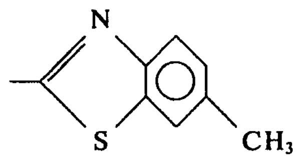 | desgl. |
| 41 | $N-(CH_2)_3-OCH_3$ | desgl. | desgl. |
| 42 | $N-CH_3$ | 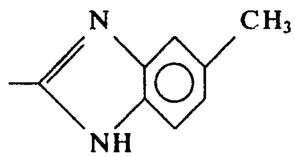 | desgl. |
| 43 | $N-(CH_2)_3-OCH_3$ | desgl. | desgl. |
| 44 | $N-CH_3$ | 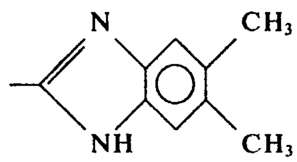 | desgl. |
| 45 | $N-(CH_2)_3-OCH_3$ | desgl. | desgl. |
| 46 | $N-CH_3$ | 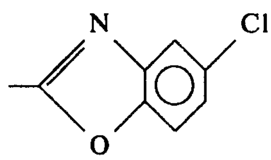 | desgl. |
| 47 | $N-(CH_2)_3-OCH_3$ | desgl. | desgl. |
| 48 | $N-CH_3$ | 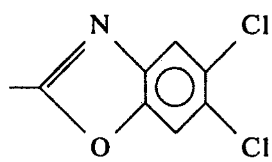 | desgl. |
| 49 | $N-(CH_2)_3-OCH_3$ | desgl. | desgl. |
| 50 | $N-CH_3$ | 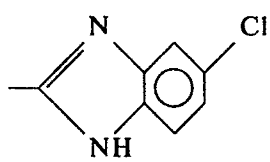 | desgl. |
| 51 | $N-(CH_2)_3-OCH_3$ | desgl. | desgl. |

Fortsetzung

| Beispiel Nr. | A | Y | Farbton auf Polyesterfasern |
|---|---|---|---|
| 52 | $N—CH_3$ | 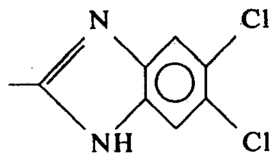 | brillantes Gelb |
| 53 | $N—(CH_2)_3—OCH_3$ | desgl. | desgl. |
| 54 | $N—CH_3$ | 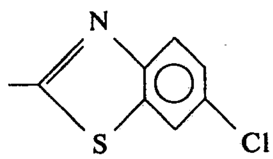 | desgl. |
| 55 | $N—(CH_2)_3—OCH_3$ | desgl. | desgl. |
| 56 | $N—CH_3$ | 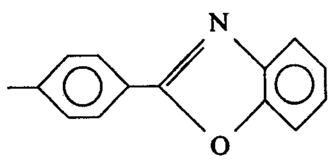 | brillantes Grüngelb |
| 57 | $N—(CH_2)_3—OCH_3$ | desgl. | desgl. |
| 58 | $N—CH_3$ | 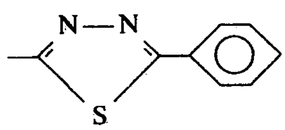 | desgl. |
| 59 | $N—(CH_2)_3—OCH_3$ | desgl. | desgl. |
| 60 | $N—CH_3$ | 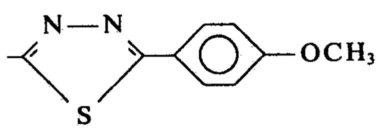 | brillantes Gelb |
| 61 | $N—(CH_2)_3—OCH_3$ | desgl. | desgl. |
| 62 | $N—CH_3$ | 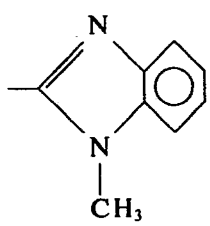 | desgl. |
| 63 | $N—(CH_2)_3—OCH_3$ | desgl. | desgl. |
| 64 | $N—CH_3$ | 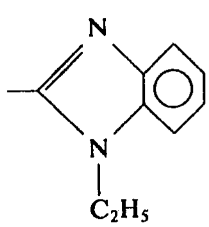 | desgl. |
| 65 | $N—(CH_2)_3—OCH_3$ | desgl. | desgl. |

Fortsetzung

| Beispiel Nr. | A | Y | Farbton auf Polyesterfasern |
|---|---|---|---|
| 66 | $N-CH_3$ | 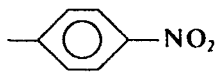 | Grüngelb |
| 67 | $N-(CH_2)_3-OCH_3$ | 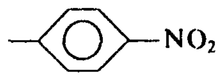 | desgl. |
| 68 | N-Stearyl | 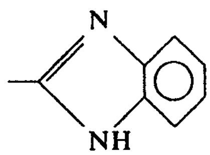 | brillantes Gelb |

Beispiel 69

255 Teile 3-Oxi-4-formyl-N-methylnaphthalimid und 129 Teile Benzylcyanid werden in 3000 Teilen Methanol suspendiert. Unter Rühren werden 370 Teile 30%ige methanolische Natriummethanolat-Lösung zugegeben und die erhaltene Mischung 24 Stunden am Rückfluß erhitzt. Das Reaktionsgemisch wird bei 0–5°C mit 1000 Teilen Wasser verdünnt und mit konzentrierter Salzsäure auf pH 8 gestellt. Das ausgefallene Produkt wird abgesaugt, mit Wasser chloridfrei gewaschen und bei 50°C im Vakuum getrocknet. Gewonnen werden 259 Teile (73% der Theorie) gelbes Farbstoffpulver der Formel

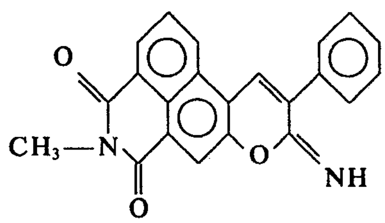

das auf Polyesterfasermaterialien grünlich gelbe Färbungen ergibt.

Beispiel 70

255 Teile 3-Oxi-4-formyl-N-methylnaphthalimid und 130 Teile 2-Pyridylacetonitril werden in 2000 Teilen Methanol suspendiert, unter Rühren mit 80 Teilen Ätzkali versetzt und 90 Stunden am Rückfluß erhitzt. Das Reaktionsgemisch wird bei 0–5°C mit 1000 Teilen Wasser verdünnt und mit konzentrierter Salzsäure auf pH 8 gestellt. Das ausgefallene Produkt wird abgesaugt, mit Wasser chloridfrei gewaschen und bei 50°C im Vakuum getrocknet. Gewonnen werden 290 Teile (82% der Theorie) gelbes Farbstoffpulver der Formel

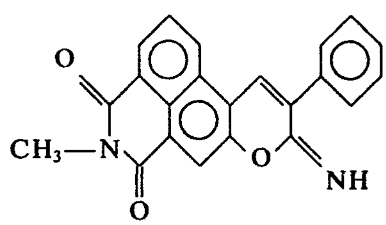

das auf Polyesterfasermaterialien grünlich gelbe Färbungen ergibt.

Verfährt man analog den Beispielen 69 und 70, so erhält man weitere erfindungsgemäße Farbstoffe, die in der nachstehenden Tabelle aufgeführt sind:

Allgemeine Formel der Farbstoffe der nachstehenden Tabellenbeispiele 70–89

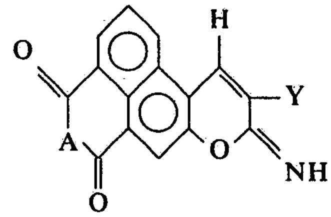

| Beispiel Nr. | A | Y | Farbton auf Polyester-fasern |
|---|---|---|---|
| 71 | $N-(CH_2)_3-OCH_3$ | 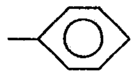 | Grünlichgelb |
| 72 | $N-(CH_2)_3-OCH_3$ | 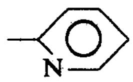 | desgl. |
| 73 | $N-CH_3$ | 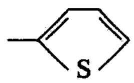 | brillantes Gelb |
| 74 | $N-(CH_2)_3-OCH_3$ | desgl. | desgl. |
| 75 | 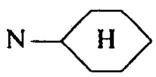 | desgl. | desgl. |
| 76 | O | desgl. | desgl. |
| 77 | $N-CH_3$ | 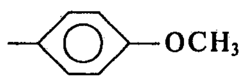 | brillantes Grüngelb |
| 78 | $N-(CH_2)_3-OCH_3$ | desgl. | desgl. |
| 79 | $N-CH_3$ | 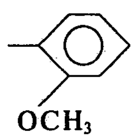 | desgl. |
| 80 | $N-(CH_2)_3-OCH_3$ | desgl. | desgl. |
| 81 | $N-CH_3$ | 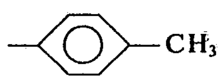 | desgl. |
| 82 | $N-(CH_2)_3-OCH_3$ | desgl. | desgl. |
| 83 | $N-CH_3$ | 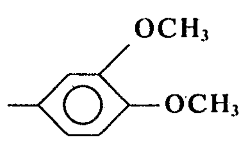 | brillantes Gelb |
| 84 | $N-(CH_2)_3-OCH_3$ | desgl. | desgl. |
| 85 | $N-CH_3$ | 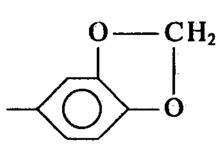 | desgl. |

Fortsetzung

| Beispiel Nr. | A | Y | Farbton auf Polyesterfasern |
|---|---|---|---|
| 86 | $N—(CH_2)_3—OCH_3$ | 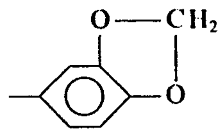 | brillantes Gelb |
| 87 | $N—CH_3$ | 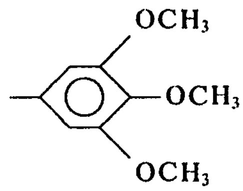 | brillantes Goldgelb |
| 88 | $N—(CH_2)_3—OCH_3$ | desgl. | desgl. |
| 89 | 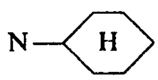 | desgl. | desgl. |

Beispiel 90

39,4 Teile der nach Beispiel 1 gewonnenen Verbindung werden in 250 Teilen Pyridin suspendiert und bei 20°C tropfenweise mit 33,3 Teilen Stearylchlorid versetzt. Man rührt 1 Stunde bei Raumtemperatur und 2 Stunden bei 50°C nach. Das ausgefallene Produkt wird nach Erkalten des Reaktionsgemisches abgesaugt, mit heißem Wasser und Äthanol gewaschen und getrocknet. Man erhält 42,9 Teile (62% der Theorie) gelben Farbstoff der Formel

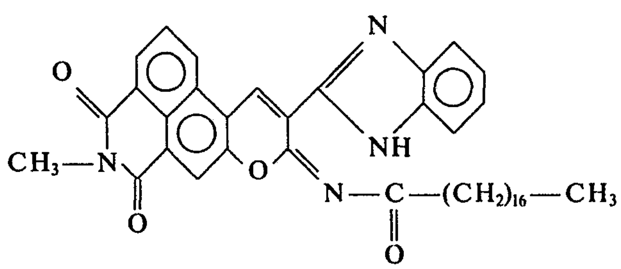

der auf Polyesterfasermaterialien brillante Gelbfärbungen ergibt.

Beispiel 91

42,3 Teile des nach Beispiel 44 erhaltenen Farbstoffes werden in 250 Teilen Pyridin suspendiert. In diese Suspension werden bei Raumtemperatur 20,9 Teile 4-Toluolsulfonsäurechlorid eingetragen und 5 Stunden bei Raumtemperatur nachgerührt. Das ausgefallene Reaktionsprodukt wird abgesaugt, mit Äthanol gewaschen und getrocknet. Man erhält 50,7 Teile (88% der Theorie) gelben Farbstoff der Formel

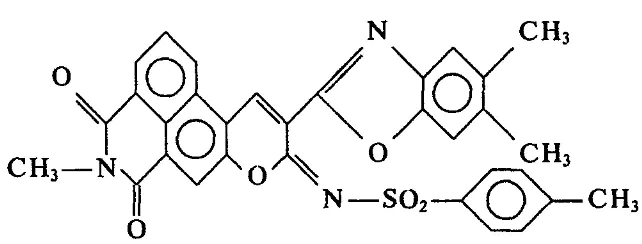

der auf Polyesterfasermaterialien brillante Gelbfärbungen ergibt.

## Beispiel 92

42,3 Teile des nach Beispiel 44 erhaltenen Farbstoffes werden in 400 Teilen Dimethylformamid bei 20°C suspendiert und nacheinander mit 11,1 Teilen Triäthylamin und 15 Teilen Chlorameisensäure-n-butylester versetzt. Man rührt 3 Stunden bei 40°C nach und saugt nach Abkühlen des Reaktionsgutes das ausgefallene Produkt ab, das mit Äthanol gewaschen und anschließend getrocknet wird. Man erhält 35,6 Teile (68% der Theorie) eines gelben Farbstoffs der Formel

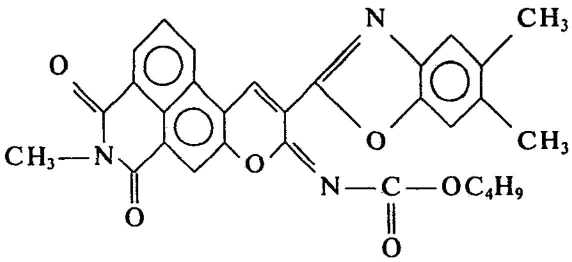

der auf Polyesterfasermaterialien brillante Gelbfärbungen ergibt.

## Beispiel 93

44,4 Teile des nach Beispiel 87 hergestellten Farbstoffes werden mit 250 Teilen Essigsäureanhydrid und 10,9 Teilen Natriumacetat 3 Stunden auf 135°C erhitzt. Nach dem Abkühlen auf Raumtemperatur wird der ausgefallene Farbstoff abgesaugt, mit Wasser neutral gewaschen und anschließend getrocknet. Man erhält 41,8 Teile (86% der Theorie) eines orangefarbenen Farbstoffs der Formel

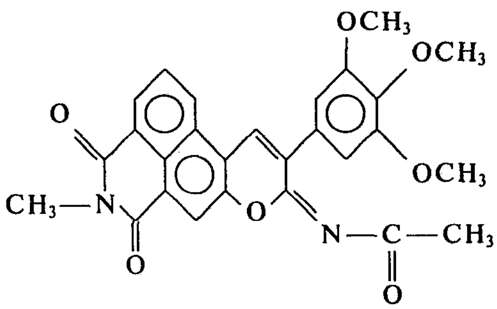

der auf Polyesterfasermaterialien brillante, goldgelbe Färbungen ergibt.

Verfährt man analog den Beispielen 90–93, so erhält man weitere erfindungsgemäße Farbstoffe, die in der nachfolgenden Tabelle aufgeführt sind.

Allgemeine Formel der Farbstoffe der nachstehenden Tabellenbeispiele 94–140

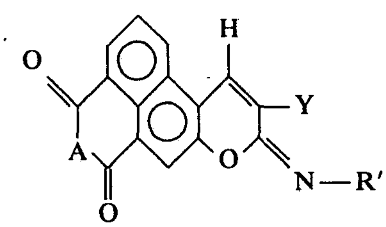

| Beispiel Nr. | A | Y | R' | Farbton auf Polyester-fasern |
|---|---|---|---|---|
| 94 | $N-(CH_2)_3-OCH_3$ | 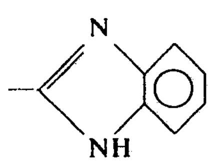 | 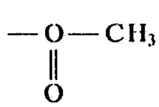 | brillantes Gelb |

Fortsetzung

| Beispiel Nr. | A | Y | R' | Farbton auf Polyester-fasern |
|---|---|---|---|---|
| 95 | $N-(CH_2)_3-OCH_3$ | 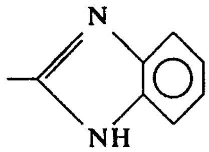 | 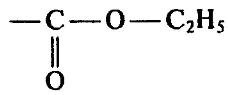 | brillantes Gelb |
| 96 | desgl. | desgl. | $-SO_2CH_3$ | desgl. |
| 97 | desgl. | desgl. | 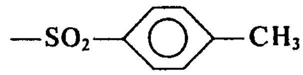 | desgl. |
| 98 | desgl. | desgl. | 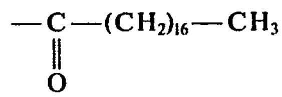 | desgl. |
| 99 | desgl. | desgl. | 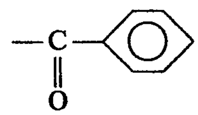 | desgl. |
| 100 | desgl. | desgl. | 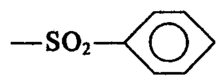 | desgl. |
| 101 | $N-CH_3$ | desgl. | 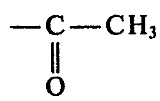 | desgl. |
| 102 | $N-(CH_2)_3-OCH_3$ | 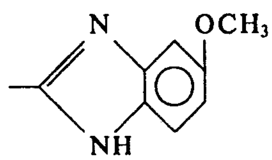 | 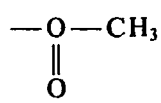 | brillantes Orange |
| 103 | $N-CH_3$ | desgl. | desgl. | desgl. |
| 104 | $N-(CH_2)_3-OCH_3$ | 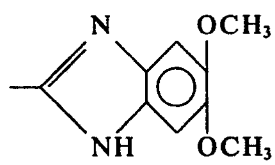 | desgl. | Rot |
| 105 | $N-CH_3$ | desgl. | desgl. | desgl. |
| 106 | $N-(CH_2)_3-OCH_3$ | 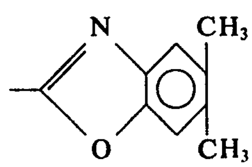 | desgl. | brillantes Gelb |
| 107 | desgl. | desgl. | 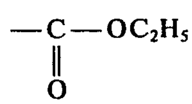 | desgl. |

Fortsetzung

| Beispiel Nr. | A | Y | R′ | Farbton auf Polyester-fasern |
|---|---|---|---|---|
| 108 | $N-(CH_2)_3-OCH_3$ | 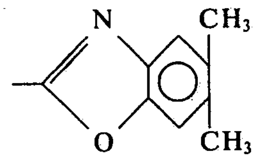 | $-\underset{\underset{O}{\Vert}}{C}-(CH_2)_{16}CH_3$ | brillantes Gelb |
| 109 | desgl. | desgl. | $-SO_2CH_3$ | desgl. |
| 110 | desgl. | desgl. | 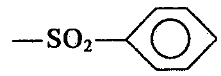 | desgl. |
| 111 | desgl. | desgl. | $-\underset{\underset{O}{\Vert}}{C}-OC_4H_9$ | desgl. |
| 112 | desgl. | desgl. | 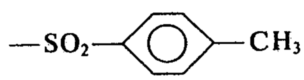 | desgl. |
| 113 | $N-CH_3$ | desgl. | $-\underset{\underset{O}{\Vert}}{C}-CH_3$ | desgl. |
| 114 | desgl. | desgl. | $-\underset{\underset{O}{\Vert}}{C}-OC_2H_5$ | desgl. |
| 115 | desgl. | desgl. | $-\underset{\underset{O}{\Vert}}{C}-(CH_2)_{16}CH_3$ | desgl. |
| 116 | desgl. | desgl. | $-SO_2CH_3$ | desgl. |
| 117 | desgl. | desgl. | 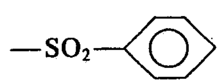 | desgl. |
| 118 | desgl. | desgl. | $-\underset{\underset{O}{\Vert}}{C}-(CH_2)_2-CH_3$ | desgl. |
| 119 | O | desgl. | $-\underset{\underset{O}{\Vert}}{O}-CH_3$ | desgl. |
| 120 | $N-(CH_2)_3-OCH_3$ | 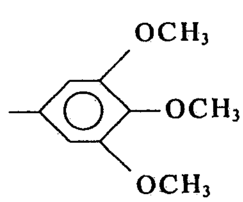 | desgl. | brillantes Goldgelb |
| 121 | desgl. | desgl. | $-\underset{\underset{O}{\Vert}}{C}-CH_2-CH_3$ | desgl. |

Fortsetzung

| Beispiel Nr. | A | Y | R' | Farbton auf Polyesterfasern |
|---|---|---|---|---|
| 122 | $N-(CH_2)_3-OCH_3$ | 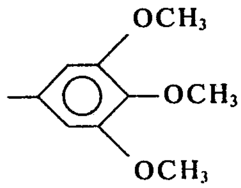 | 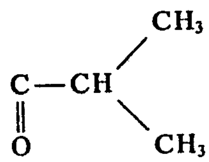 | brillantes Goldgelb |
| 123 | desgl. | desgl. | 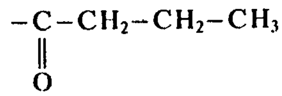 | desgl. |
| 124 | desgl. | desgl. | 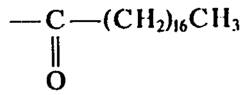 | desgl. |
| 125 | desgl. | desgl. | 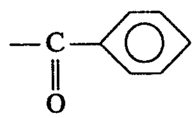 | desgl. |
| 126 | desgl. | desgl. | 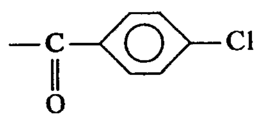 | desgl. |
| 127 | desgl. | desgl. | $—SO_2—CH_3$ | desgl. |
| 128 | desgl. | desgl. | 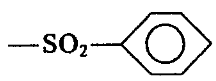 | desgl. |
| 129 | desgl. | desgl. | 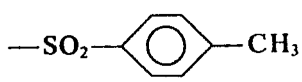 | desgl. |
| 130 | desgl. | desgl. | 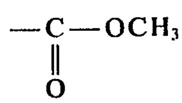 | desgl. |
| 131 | desgl. | desgl. | 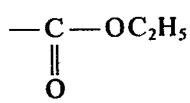 | desgl. |
| 132 | desgl. | desgl. | 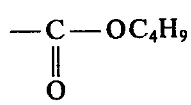 | desgl. |
| 133 | $N—CH_3$ | desgl. | 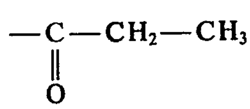 | desgl. |
| 134 | desgl. | desgl. | 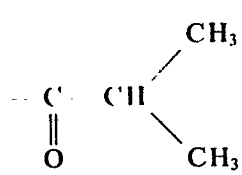 | desgl. |

Fortsetzung

| Beispiel Nr. | A | Y | R' | Farbton auf Polyesterfasern |
|---|---|---|---|---|
| 135 | $N-CH_3$ | 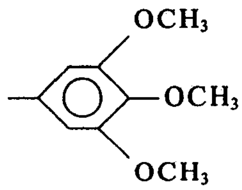 | 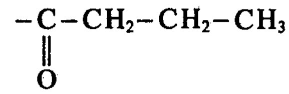 | brillantes Goldgelb |
| 136 | desgl. | desgl. | 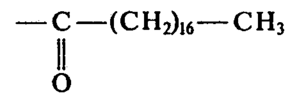 | desgl. |
| 137 | desgl. | desgl. | 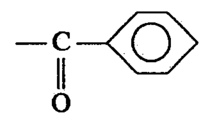 | desgl. |
| 138 | desgl. | desgl. | $-SO_2-CH_3$ | desgl. |
| 139 | desgl. | desgl. | 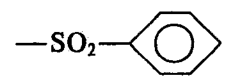 | desgl. |
| 140 | desgl. | desgl. | 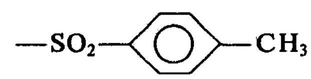 | desgl. |

Beispiel 141

39,4 Teile des nach Beispiel 1 gewonnenen Farbstoffs werden in 200 Teilen Anilin 2 Stunden bei 140°C gerührt. Bei Raumtemperatur wird das Reaktionsprodukt mit 200 Teilen Äthanol ausgerührt, abgesaugt, mit Äthanol gewaschen und getrocknet. Man erhält 36,7 Teile (78% der Theorie) rotes Farbstoffpulver der Formel

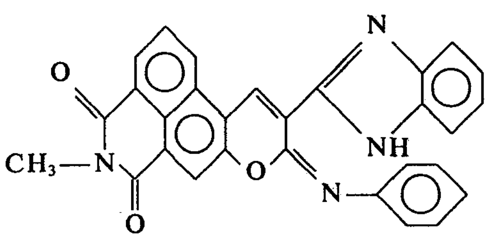

das auf Polyesterfasermaterialien leuchtende Orangefärbungen ergibt.

Beispiel 142

39,4 Teile des nach Beispiel 1 gewonnenen Farbstoffs und 12,2 Teile 4-Methoxyanilin werden in 250 Teilen Dimethylformamid 3 Stunden bei 140°C gerührt. Bei Raumtemperatur wird das ausgefallene Produkt abgesaugt, mit Äthanol gewaschen und getrocknet. Man erhält 42,9 Teile (86% der Theorie) rotes Farbstoffpulver der Formel

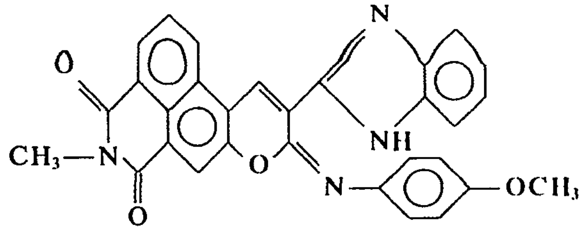

das auf Polyesterfasermaterialien Orangefärbungen ergibt.

Beispiel 143

42,3 Teile des nach Beispiel 31 gewonnenen Farbstoffs werden in 300 Teilen Anilin $2^1/_2$ Stunden bei 140°C gerührt. Bei Raumtemperatur rührt man 300 Teile Äthanol ein und saugt das ausgefallene Produkt ab, das mit Äthanol gewaschen und anschließend getrocknet wird. Man erhält 43,9 Teile (83% der Theorie) roten Farbstoff der Formel

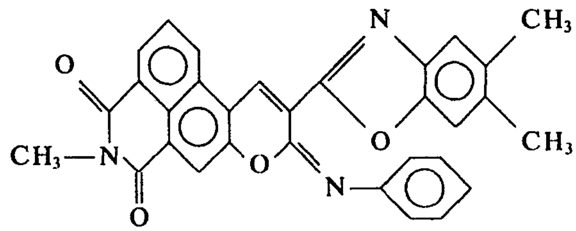

das auf Polyesterfasermaterialien Orangefärbungen ergibt.

Verfährt man analog den Beispielen 141—143, so erhält man weitere erfindungsgemäße Farbstoffe, die in der folgenden Tabelle aufgeführt sind:

Allgemeine Formel der Farbstoffe der nachstehenden Tabellenbeispiele 144—156

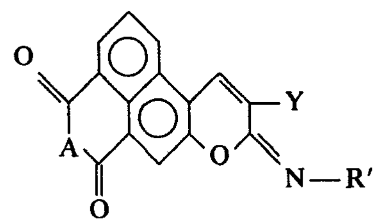

| Beispiel Nr. | A | Y | R' | Farbton auf Polyester-fasern |
|---|---|---|---|---|
| 144 | $N-(CH_2)_3-OCH_3$ | 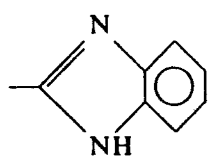 | 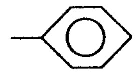 | Orange |
| 145 | desgl. | desgl. | 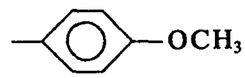 | desgl. |
| 146 | desgl. | desgl. | 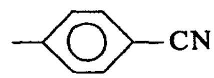 | desgl. |
| 147 | $N-CH_3$ | desgl. | 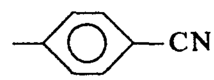 | desgl. |
| 148 | desgl. | 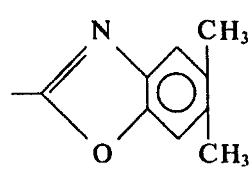 | 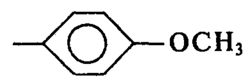 | Rot |

Fortsetzung

| Beispiel Nr. | A | Y | R' | Farbton auf Polyester-fasern |
|---|---|---|---|---|
| 149 | $N-CH_3$ | 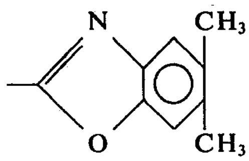 | 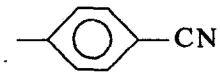 | Orange |
| 150 | $N-(CH_2)_3-OCH_3$ | desgl. | 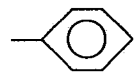 | desgl. |
| 151 | desgl. | desgl. | 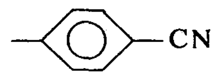 | desgl. |
| 152 | desgl. | desgl. | 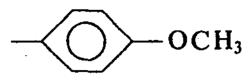 | Rot |
| 153 | desgl. | 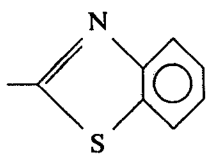 | 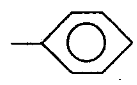 | Orange |
| 154 | desgl. | 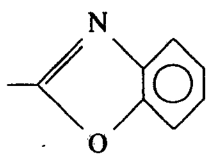 | desgl. | rotstichiges Gelb |
| 155 | desgl. | 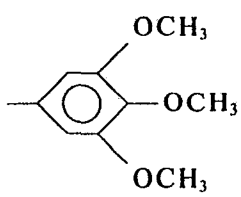 | desgl. | desgl. |
| 156 | $CH_3$ | desgl. | 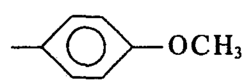 | desgl. |

Beispiel 157

39,5 Teile des nach Beispiel 21 gewonnenen Farbstoffs werden in 250 Teilen Chlorbenzol suspendiert, mit 13,1 Teilen Phenylisocyanat versetzt und 18 Stunden bei 80°C gerührt. Nach dem Erkalten wird das Reaktionsprodukt abgesaugt, mit Äthanol gewaschen und getrocknet. Man erhält 47,3 Teile (92% der Theorie) gelben Farbstoff der Formel

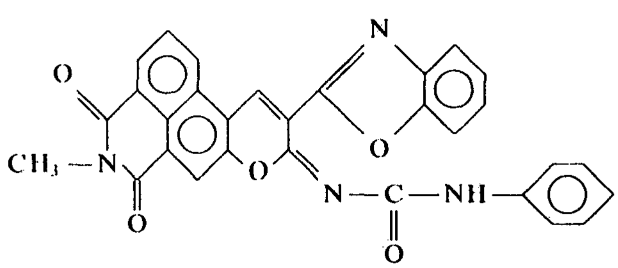

der auf Polyesterfasermaterialien grüngelbe Färbungen ergibt.

## Beispiel 158

48,1 Teile des nach Beispiel 32 gewonnenen Farbstoffs werden in 400 Teilen Dioxan suspendiert, mit 13,1 Teilen Phenylisocyanat versetzt und 20 Stunden am Rückfluß erhitzt. Nach dem Erkalten wird das Reaktionsprodukt abgesaugt, mit Äthanol gewaschen und getrocknet. Man erhält 53,4 Teile (89% der Theorie) gelben Farbstoff der Formel

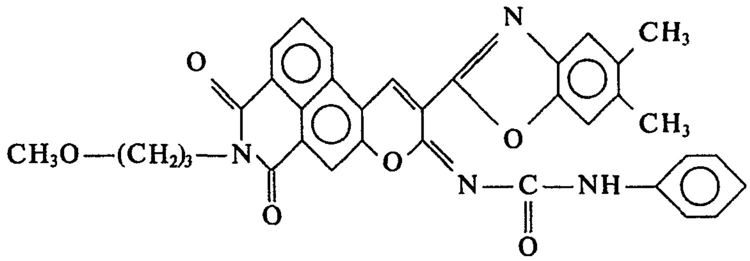

der auf Polyesterfasermaterialien brillante, gelbe Färbungen ergibt.

Verfährt man analog den Beispielen 157 und 158, so erhält man weitere erfindungsgemäße Farbstoffe, die in der folgenden Tabelle aufgeführt sind:

Allgemeine Formel der Farbstoffe der nachstehenden Tabellenbeispiele 159–167

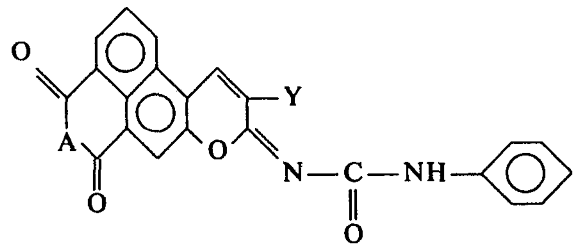

| Beispiel Nr. | A | Y | Farbton auf Polyesterfasern |
|---|---|---|---|
| 159 | N–$(CH_2)_3$–$OCH_3$ | 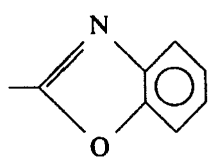 | brillantes Gelbgrün |
| 160 | 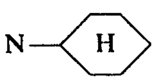 | desgl. | desgl. |
| 161 | N–$CH_3$ | 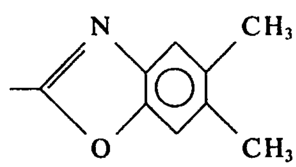 | brillantes Gelb |
| 162 | 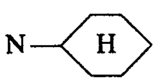 | desgl. | desgl. |
| 163 | N–$CH_3$ | 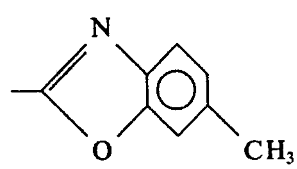 | desgl. |
| 164 | N—$(CH_2)_3$—$OCH_3$ | desgl. | desgl. |

Fortsetzung

| Beispiel Nr. | A | Y | Farbton auf Polyesterfasern |
|---|---|---|---|
| 165 | $N—CH_3$ | 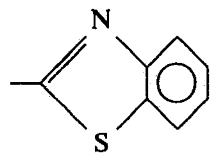 | brillantes Gelb |
| 166 | $N—(CH_2)_3—OCH_3$ | desgl. | desgl. |
| 167 | $N—CH_3$ | 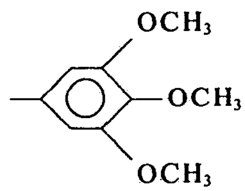 | brillantes Goldgelb |

Beispiel 168

255 Teile 3-Oxi-4-formyl-N-methylnaphthalimid und 207 Teile Benzthiazol-2-essigsäuremethylester werden mit 10 Teilen Piperidinacetat in 6000 Teilen Toluol und 1000 Teilen Dimethylformamid 4 Stunden am Wasserabscheider zum Rückfluß erhitzt. Das Reaktionsprodukt wird bei 0°C abgesaugt, mit Äthanol gewaschen und getrocknet. Man erhält 395,5 Teile (96% der Theorie) gelben Farbstoff der Formel

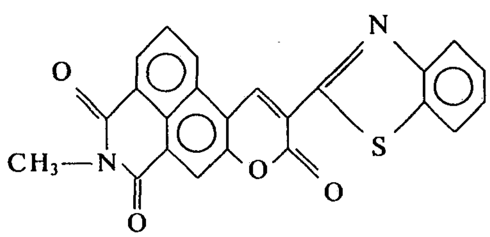

der auf Polyesterfasermaterialien brillante Gelbfärbungen ergibt.

Beispiel 169

255 Teile 3-Oxi-4-formyl-N-methylnaphthalimid werden mit 5 Teilen Piperidinacetat in 3000 Teilen Diäthylmalonat 4 Stunden unter destillativer Entfernung der flüchtigen Reaktionsprodukte bei 130°C gerührt. Nach Abkühlen auf Raumtemperatur wird das Reaktionsprodukt abgesaugt, mit Äthanol gewaschen und getrocknet. Man erhält 301,9 Teile (86% der Theorie) gelben Farbstoff der Formel

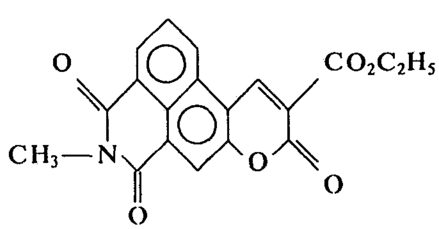

der auf Polyesterfasermaterialien grün-gelbe Färbungen ergibt.

Beispiel 170

452 Teile des nach Beispiel 2 hergestellten Farbstoffs werden mit 6000 Teilen 5%iger Salzsäure

3 Stunden am Rückfluß erhitzt. Nach Erkalten auf Raumtemperatur wird das Reaktionsprodukt abgesaugt, mit Wasser chloridfrei gewaschen und getrocknet. Man erhält 448,5 Teile (99% der Theorie) eines orangefarbenen Farbstoffpulvers der Formel

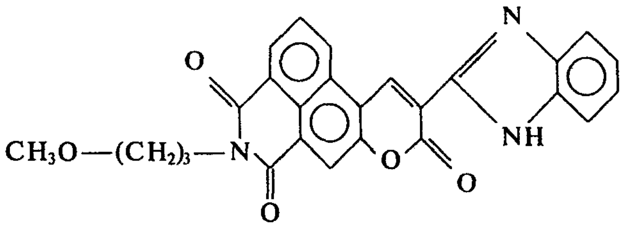

das auf Polyesterfasermaterialien brillante gelbe Färbungen ergibt.

Beispiel 171

255 Teile 3-Oxi-4-formyl-N-methylnaphthalimid und 66 Teile Malonsäuredinitril werden mit 10 Teilen Piperidin in 2500 Teilen Äthanol bei Raumtemperatur gerührt. Nach 4 Stunden werden 1000 Teile Wasser und 250 Teile konz. Salzsäure zugesetzt und 2 Stunden am Rückfluß gekocht. Das Reaktionsprodukt wird bei Raumtemperatur abgesaugt, mit Wasser chloridfrei gewaschen und getrocknet. Man erhält 237 Teile (78% der Theorie) eines gelben Farbstoffpulvers der Formel

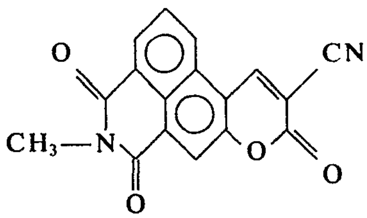

das auf Polyesterfasermaterialien grünlich gelbe Färbungen ergibt.

Verfährt man analog den Beispielen 168—171, so erhält man weitere erfindungsgemäße Farbstoffe, die in der folgenden Tabelle aufgeführt sind:

Allgemeine Formel der Farbstoffe der nachstehenden Tabellenbeispiele 172—256

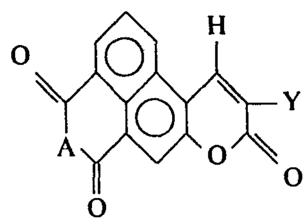

| Beispiel Nr. | A | Y | Farbton auf Polyesterfasern |
|---|---|---|---|
| 172 | N-$CH_3$ | 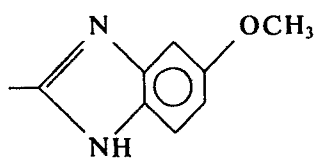 | brillantes Orange |
| 173 | N 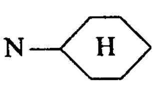 | desgl. | desgl. |
| 174 | N-$CH_3$ | 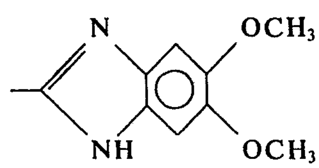 | Rot |

Fortsetzung

| Beispiel Nr. | A | Y | Farbton auf Polyesterfasern |
|---|---|---|---|
| 175 | 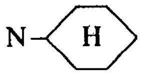 | 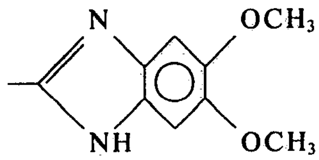 | Rot |
| 176 | O | desgl. | desgl. |
| 177 | $N-CH_3$ | 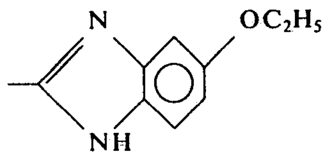 | brillantes Orange |
| 178 | $N-(CH_2)_3-OCH_3$ | desgl. | desgl. |
| 179 | O | desgl. | desgl. |
| 180 | 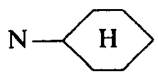 | desgl. | desgl. |
| 181 | $N-CH_3$ | 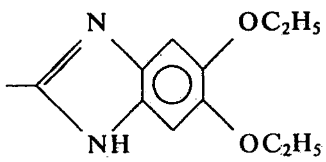 | Rot |
| 182 | $N-(CH_2)_3-OCH_3$ | desgl. | desgl. |
| 183 | $N-CH_3$ | 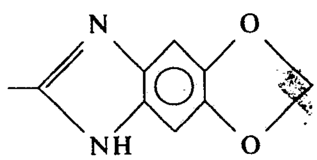 | desgl. |
| 184 | $N-(CH_2)_3-OCH_3$ | desgl. | desgl. |
| 185 | $N-CH_3$ | 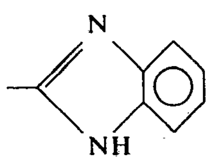 | brillantes Gelb |
| 186 | 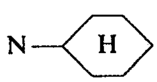 | desgl. | desgl. |
| 187 | $N-CH_3$ | 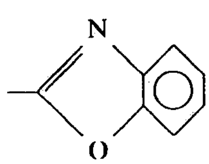 | brillantes Grüngelb |
| 188 | $N-(CH_2)_3-OCH_3$ | desgl. | desgl. |
| 189 | O | desgl. | desgl. |

Fortsetzung

| Beispiel Nr. | A | Y | Farbton auf Polyesterfasern |
|---|---|---|---|
| 190 | 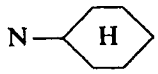 | 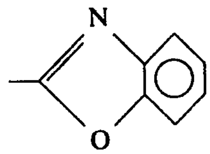 | brillantes Grüngelb |
| 191 | $N-CH_3$ | 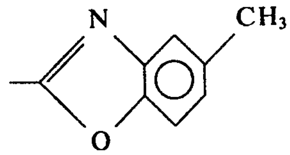 | desgl. |
| 192 | $N-(CH_2)_3-OCH_3$ | desgl. | desgl. |
| 193 | $N-CH_3$ | 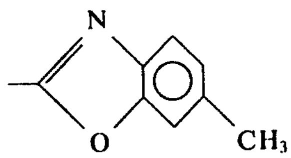 | brillantes Gelb |
| 194 | $N-(CH_2)_3-OCH_3$ | desgl. | desgl. |
| 195 | $N-CH_3$ | 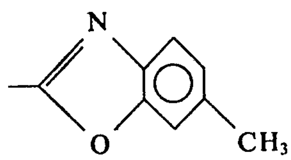 | desgl. |
| 196 | $N-(CH_2)_3-OCH_3$ | desgl. | desgl. |
| 197 | $N-CH_3$ | 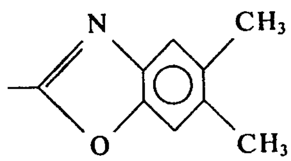 | desgl. |
| 198 | $N-(CH_2)_3-OCH_3$ | desgl. | desgl. |
| 199 | 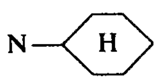 | desgl. | desgl. |
| 200 | $N-CH_3$ | 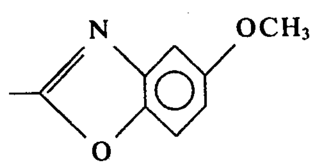 | desgl. |
| 201 | $N-(CH_2)_3-OCH_3$ | desgl. | desgl. |
| 202 | $N-(CH_2)_3-OCH_3$ | $-CO_2C_2H_5$ | Grüngelb |

Fortsetzung

| Beispiel Nr. | A | Y | Farbton auf Polyesterfasern |
|---|---|---|---|
| 203 | $N—(CH_2)_3—OCH_3$ | 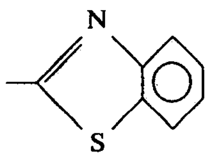 | brillantes Gelb |
| 204 | $N—CH_3$ | 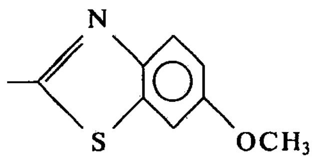 | desgl. |
| 205 | $N—(CH_2)_3—OCH_3$ | desgl. | desgl. |
| 206 | $N—CH_3$ | 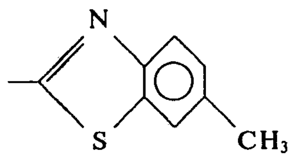 | desgl. |
| 207 | $N—(CH_2)_3—OCH_3$ | desgl. | desgl. |
| 208 | $N—CH_3$ | 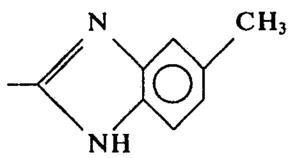 | desgl. |
| 209 | $N—(CH_2)_3—OCH_3$ | desgl. | desgl. |
| 210 | $N—CH_3$ | 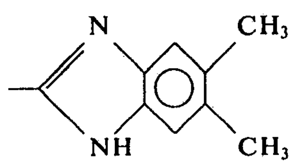 | desgl. |
| 211 | $N—(CH_2)_3—OCH_3$ | desgl. | desgl. |
| 212 | $N—CH_3$ | 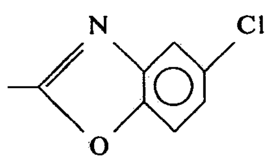 | desgl. |
| 213 | $N—(CH_2)_3—OCH_3$ | desgl. | desgl. |
| 214 | $N—CH_3$ | 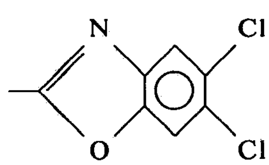 | desgl. |
| 215 | $N—(CH_2)_3—OCH_3$ | desgl. | desgl. |

Fortsetzung

| Beispiel Nr. | A | Y | Farbton auf Polyesterfasern |
|---|---|---|---|
| 216 | N—$CH_3$ | 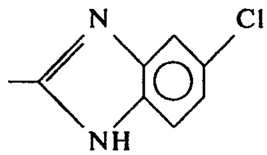 | brillantes Gelb |
| 217 | N—$(CH_2)_3$—$OCH_3$ | desgl. | desgl. |
| 218 | N—$CH_3$ | 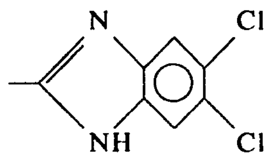 | desgl. |
| 219 | N—$(CH_2)_3$—$OCH_3$ | desgl. | desgl. |
| 220 | N—$CH_3$ | 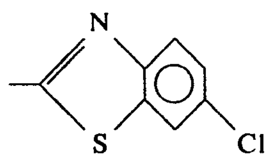 | desgl. |
| 221 | N—$(CH_2)_3$—$OCH_3$ | desgl. | desgl. |
| 222 | N—$CH_3$ | 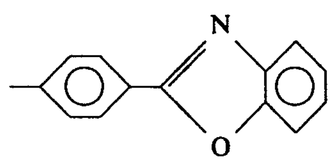 | brillantes Grüngelb |
| 223 | N—$(CH_2)_3$—$OCH_3$ | desgl. | desgl. |
| 224 | N—$CH_3$ | 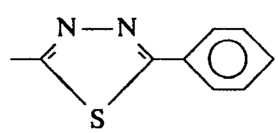 | desgl. |
| 225 | N—$(CH_2)_3$—$OCH_3$ | desgl. | desgl. |
| 226 | N—$CH_3$ | 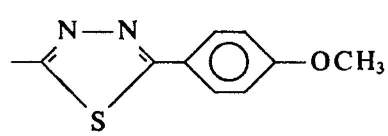 | brillantes Gelb |
| 227 | N—$(CH_2)_3$—$OCH_3$ | desgl. | desgl. |
| 228 | N—$CH_3$ | 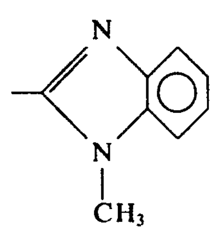 | desgl. |

Fortsetzung

| Beispiel Nr. | A | Y | Farbton auf Polyesterfasern |
|---|---|---|---|
| 229 | $N-(CH_2)_3-OCH_3$ | 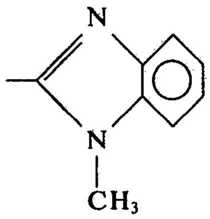 | brillantes Gelb |
| 230 | $N-CH_3$ | 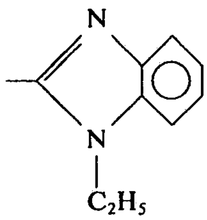 | desgl. |
| 231 | $N-(CH_2)_3-OCH_3$ | desgl. | desgl. |
| 232 | $N-CH_3$ | 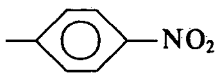 | Grüngelb |
| 233 | $N-(CH_2)_3-OCH_3$ | desgl. | desgl. |
| 234 | $N-(CH_2)_3-OCH_3$ | 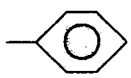 | Grünlichgelb |
| 235 | $N-(CH_2)_3-OCH_3$ | 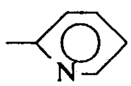 | desgl. |
| 236 | $N-CH_3$ | 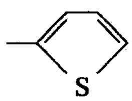 | brillantes Gelb |
| 237 | $N-(CH_2)_3-OCH_3$ | desgl. | desgl. |
| 238 | 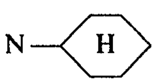 | desgl. | desgl. |
| 239 | O | desgl. | desgl. |
| 240 | $N-CH_3$ | 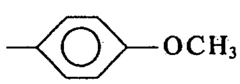 | brillantes Grüngelb |
| 241 | $N-(CH_2)_3-OCH_3$ | desgl. | desgl. |
| 242 | $N-CH_3$ | 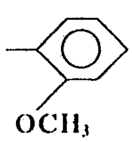 | desgl. |
| 243 | $N-(CH_2)_3-OCH_3$ | desgl. | desgl. |
| 244 | $N-CH_3$ | 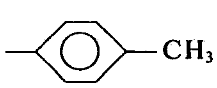 | desgl. |

Fortsetzung

| Beispiel Nr. | A | Y | Farbton auf Polyesterfasern |
|---|---|---|---|
| 245 | $N-(CH_2)_3-OCH_3$ | 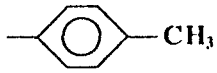 | brillantes Grüngelb |
| 246 | $N-CH_3$ | 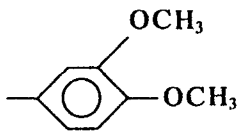 | brillantes Gelb |
| 247 | $N-(CH_2)_3-OCH_3$ | desgl. | desgl. |
| 248 | $N-CH_3$ | 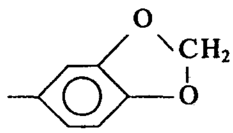 | desgl. |
| 249 | $N-(CH_2)_3-OCH_3$ | desgl. | desgl. |
| 250 | $N-CH_3$ | 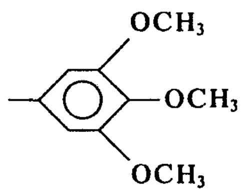 | brillantes Goldgelb |
| 251 | $N-(CH_2)_3-OCH_3$ | desgl. | desgl. |
| 252 | N— 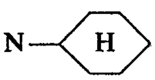 | desgl. | desgl. |
| 253 | $N-(CH_2)_3-OCH_3$ | $-CN$ | Grünlichgelb |
| 254 | desgl. | $-CO_2C_2H_5$ | desgl. |
| 255 | desgl. | $-SO_2-$ 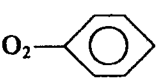 | brillantes Grüngelb |
| 256 | $N-CH_3$ | desgl. | desgl. |

## Beispiel 257

77 Teile des nach Beispiel 240 hergestellten Farbstoffs werden in 500 Teilen Dimethylformamid suspendiert und bei Raumtemperatur tropfenweise mit 60 Teilen 30%iger Kaliumcyanid-Lösung versetzt. Man erhitzt 4 Stunden auf 60°C, kühlt auf –5°C ab, tropft 11,2 Teile Brom zu und führt 5 Stunden bei Raumtemperatur nach. Das erhaltene Produkt der Formel

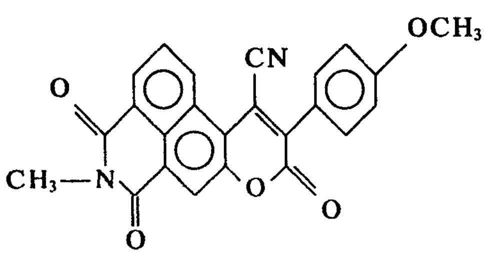

wird abgesaugt, mit Wasser bromidfrei gewaschen und getrocknet. Man erhält 56,6 Teile (69% der Theorie) eines roten Farbstoffes, der auf Polyesterfasermaterialien brillante Gelbfärbungen ergibt.

Beispiel 258

84,8 Teile des nach Beispiel 197 hergestellten Farbstoffs werden in 1000 Teilen Dimethylformamid suspendiert und bei Raumtemperatur tropfenweise mit 60 Teilen 30%iger Kaliumcyanid-Lösung versetzt. Man rührt zwei Stunden bei Raumtemperatur nach, kühlt auf −5 °C ab, tropft 11,2 Teile Brom zu und rührt 4 Stunden bei Raumtemperatur nach. Das ausgefallene Produkt der Formel

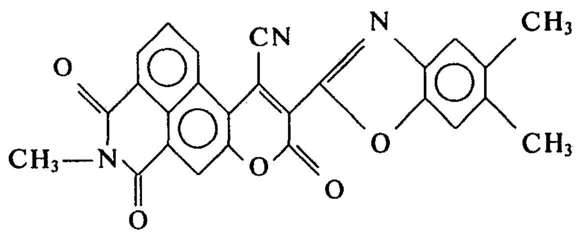

wird abgesaugt, mit Wasser bromidfrei gewaschen und getrocknet. Man erhält 68,2 Teile (76% der Theorie) eines roten Farbstoffes der auf Polyesterfasermaterialien brillante Orangefärbungen ergibt.

Verfährt man analog den Beispielen 257 und 258, so erhält man weitere erfindungsgemäße Farbstoffe, die in der folgenden Tabelle aufgeführt sind:

Allgemeine Formel der Farbstoffe der nachstehenden Tabellenbeispiele 259–277

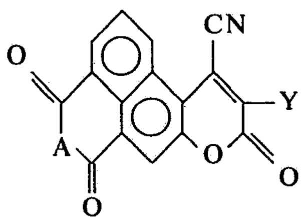

| Beispiel Nr. | A | Y | Farbton auf Polyesterfasern |
|---|---|---|---|
| 259 | $N-(CH_2)_3-OCH_3$ | 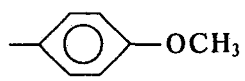 | brillantes Gelb |
| 260 | desgl. | 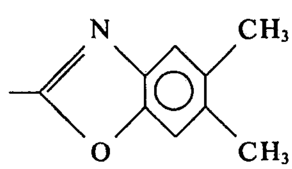 | brillantes Orange |
| 261 | $N-CH_3$ | 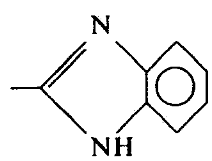 | desgl. |
| 262 | $N-(CH_2)_3-OCH_3$ | desgl. | desgl. |
| 263 | 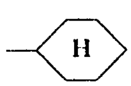 | desgl. | desgl. |
| 264 | $N-CH_3$ | 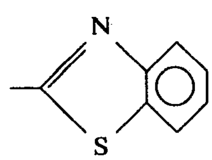 | desgl. |

Fortsetzung

| Beispiel Nr. | A | Y | Farbton auf Polyesterfasern |
|---|---|---|---|
| 265 | $N-(CH_2)_3-OCH_3$ | 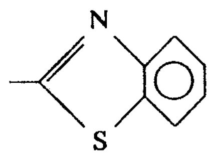 | brillantes Orange |
| 266 | $N-CH_3$ | 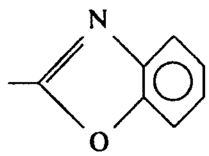 | brillantes Gelb |
| 267 | $N-(CH_2)_3-OCH_3$ | desgl. | desgl. |
| 268 | $N-CH_3$ | 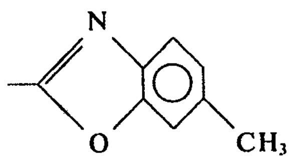 | brillantes Orange |
| 269 | $N-(CH_2)_3-OCH_3$ | desgl. | desgl. |
| 270 | $N-CH_3$ | 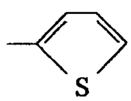 | desgl. |
| 271 | $N-(CH_2)_3-OCH_3$ | desgl. | desgl. |
| 272 | $N-CH_3$ | 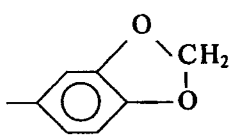 | desgl. |
| 273 | $N-(CH_2)_3-OCH_3$ | desgl. | desgl. |
| 274 | $N-CH_3$ | 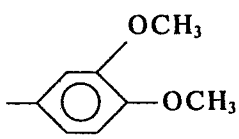 | desgl. |
| 275 | $N-(CH_2)_3-OCH_3$ | desgl. | desgl. |
| 276 | $N-CH_3$ | 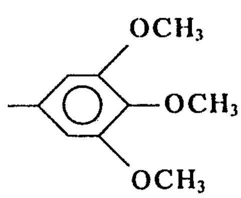 | Rot |
| 277 | $N-(CH_2)_3-OCH_3$ | desgl. | desgl. |

**Patentansprüche**

1. Cumarinverbindungen der allgemeinen Formel (1)

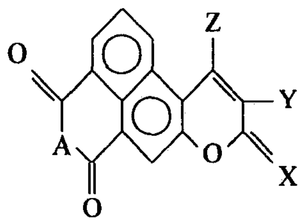

(1)

in welcher A ein Sauerstoffatom oder die Gruppierung >N—R bedeutet, worin R ein Wasserstoffatom oder einen gegebenenfalls durch Fluor-, Chlor- oder Bromatome oder Alkoxy $_{C_1-C_4}$, Dialkyl $_{C_1-C_4}$ amino-, $-SO_2-N(alkyl_{C_1-C_4})_2$- oder Cyangruppen substituierter Alkylrest von 1 bis 18 Kohlenstoffatomen, oder einen gegebenenfalls durch Fluor-, Chlor- oder Bromatome oder Alkyl $_{C_1-C_4}$, Alkoxy $_{C_1-C_4}$, Hydroxyl-, Cyan-, Sulfonamid- oder $-SO_2-N-(Alkyl_{C_1-C_4})_2$-Gruppen substituierten cycloaliphatischen, aromatischen oder heterocyclischen Rest darstellt, X ein Sauerstoffatom oder die Gruppierung >N—R' bedeutet, worin R' ein Wasserstoffatom oder eine Alkyl $_{C_1-C_{18}}$carbonyl-, Alkoxy$_{C_1-C_4}$carbonyl-, Alkyl$_{C_1-C_4}$-carbamoyl-, Alkyl$_{C_1-C_4}$sulfonylgruppe oder einen gegebenenfalls durch Fluor-, Chlor- oder Bromatome oder Alkyl- oder Alkoxygruppen von jeweils 1 bis 4 Kohlenstoffatomen oder Cyangruppen substituierten Aryl-, Arylcarbonyl-, Arylcarbamoyl- oder Arylsulfonylrest darstellt, Y ein Wasserstoffatom oder eine Cyan- oder Carbonsäurealkyl$_{C_1-C_4}$estergruppe, eine gegebenenfalls durch Fluor-, Chlor- oder Bromatome oder Alkyl- oder Alkoxygruppen von 1 bis 4 Kohlenstoffatomen, Nitro-, Methylendioxy-, Amino-, Monoalkyl$_{C_1-C_4}$amino-, Dialkyl$_{C_1-C_4}$aminogruppen oder einen Benzoxazolylrest substituierten Aryl-, Arylsulfonyl- oder —CO—O—aryl-Rest, einen gegebenenfalls quaternierten und gegebenenfalls durch Fluor-, Chlor- oder Bromatome oder Alkyl- oder Alkoxygruppen von 1 bis 4 Kohlenstoffatomen, Nitro-, Phenyl-, Alkyl$_{C_1-C_4}$phenyl-, Alkoxy$_{C_1-C_4}$phenyl-, Cyan-, Methylendioxy- oder Carbonsäurealkyl$_{C_1-C_4}$estergruppen substituierten heterocyclischen Rest, und Z ein Wasserstoffatom oder eine Carbonsäurealkyl$_{C_1-C_4}$ester- oder Cyangruppe bedeutet.

2. Cumarinverbindung der Formel

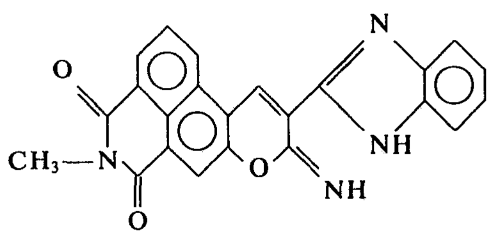

3. Cumarinverbindung der Formel

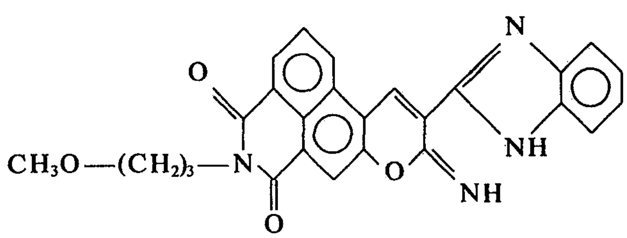

4. Cumarinverbindung der Formel

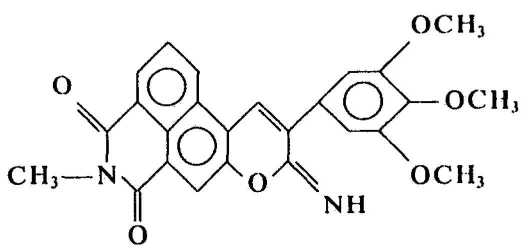

5. Cumarinverbindung der Formel

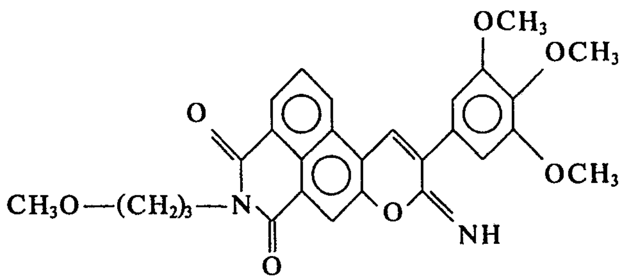

6. Cumarinverbindung der Formel

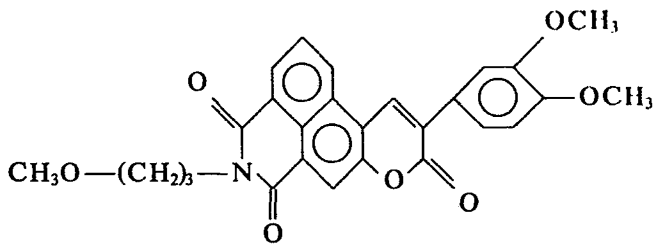

7. Cumarinverbindung der Formel

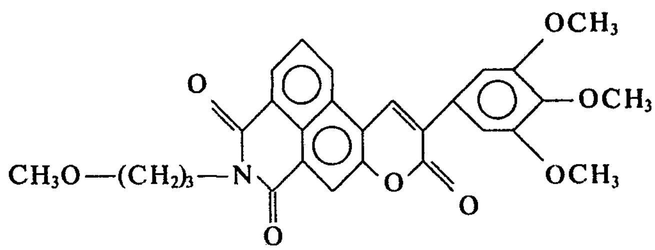

8. Verfahren zur Herstellung von Cumarinverbindungen der in Anspruch 1 genannten allgemeinen Formel (1), dadurch gekennzeichnet, daß man eine Verbindung der Formel (2)

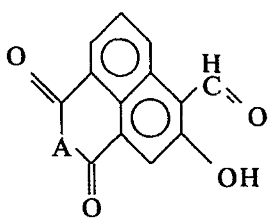

(2)

in welcher A die in Anspruch 1 genannten Bedeutungen hat,

a) mit einer Verbindung der allgemeinen Formel (3)

$$NC—CH_2—Y \quad (3)$$

in welcher Y die in Anspruch 1 genannten Bedeutungen hat, in an sich bekannter Weise kondensiert und die hierbei erhaltene Iminocumarinverbindung der allgemeinen Formel (4)

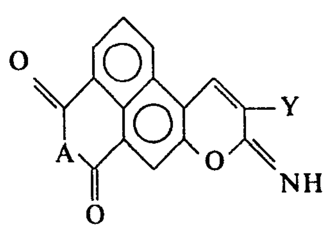

(4)

in welcher A und Y die in Anspruch 1 genannten Bedeutungen haben, nach an sich bekannten Verfahren acyliert, mit Arylaminen behandelt oder verseift, oder

b) mit einer Verbindung der allgemeinen Formel (5)

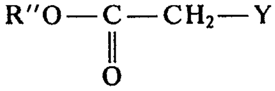
(5)

in welcher Y die in Anspruch 1 genannten Bedeutungen hat und R″ einen gegebenenfalls substituierten Alkyl- oder Arylrest bedeutet, in an sich bekannter Weise kondensiert und die hierbei erhaltene Cumarinverbindung der allgemeinen Formel (6)

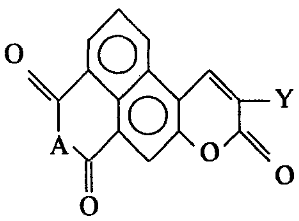
(6)

in welcher A und Y die in Anspruch 1 genannten Bedeutungen haben, gegebenenfalls nach an sich bekannten Verfahren mit einem Cyanidsalz umsetzt und die als Zwischenprodukt gebildete Anlagerungsverbindung gleichzeitig oder nachträglich mit einem Oxidationsmittel behandelt unter Erhalt der Verbindung der allgemeinen Formel (7)

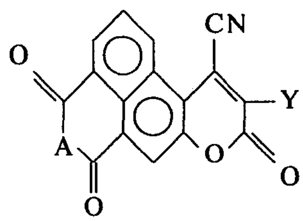
(7)

9. Verwendung der Verbindungen der in Anspruch 1 genannten allgemeinen Formel (1) als Farbstoffe.

10. Verwendung nach Anspruch 9, dadurch gekennzeichnet, daß die Farbstoffe zum Färben oder Bedrucken von synthetischen Fasermaterialien, insbesondere solchen aus Polyestern, gegebenenfalls in Mischung mit natürlichen Fasermaterialien, eingesetzt werden.

**Claims**

1. Coumarin compounds of the general formula (1)

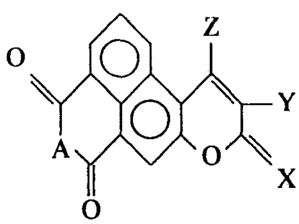
(1)

in which A denotes an oxygen atom or the grouping >N—R wherein R represents a hydrogen atom or an alkyl radical of 1 to 18 carbon atoms optionally substituted by fluorine, chlorine or bromine atoms or by alkoxy$_{C_1-C_4}$, dialkyl$_{C_1-C_4}$-amino, $-SO_2-N(alkyl_{C_1-C_4})_2$ or cyano groups; or is a cycloaliphatic, aromatic or heterocyclic radical optionally substituted by fluorine, chlorine or bromine atoms or by alkyl$_{C_1-C_4}$, alkoxy$_{C_1-C_4}$, hydroxyl, cyano, sulfonamide or $-SO_2-N(alkyl_{C_1-C_4})_2$ groups; X denotes an oxygen atom or the grouping >N—R′ wherein R′ represents a hydrogen atom or an alkyl$_{C_1-C_{18}}$carbonyl, alkoxy$_{C_1-C_4}$carbonyl, alkyl$_{C_1-C_4}$carbamoyl, or alkyl$_{C_1-C_4}$ sulfonyl group; or is an aryl, arylcarbonyl, arylcarbamoyl or arylsulfonyl radical optionally substituted by fluorine, chlorine or bromine atoms or by alkyl or alkoxy groups of 1 to 4 carbon atoms each or by cyano groups; Y denotes a hydrogen atom or a cyano or an alkyl$_{C_1-C_4}$carboxylate group; or is an aryl, arylsulfonyl or —CO—O=aryl radical optionally substituted by fluorine, chlorine or bromine atoms or by alkyl or alkoxy groups of 1 to 4 carbon atoms, or by nitro, methylenedioxy, amino, monoalkyl$_{C_1-C_4}$amino or dialkyl$_{C_1-C_4}$amino groups or by a benzoxazolyl radical; or is a heterocyclic radical optionally quaternized and optionally substituted by fluorine, chlorine or

bromine atoms or by alkyl or alkoxy groups of 1 to 4 carbon atoms, or by nitro, phenyl, alkyl$_{\overline{C_1-C_4}}$phenyl, alkoxy$_{\overline{C_1-C_4}}$phenyl, cyano, methylenedioxy or alkyl$_{\overline{C_1-C_4}}$carboxylate groups; and Z denotes a hydrogen atom or an alkyl$_{\overline{C_1-C_4}}$carboxylate or a cyano group.

2. Coumarin compound of the formula

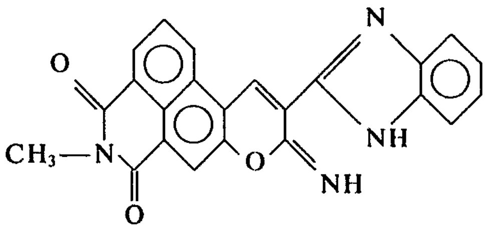

3. Coumarin compound of the formula

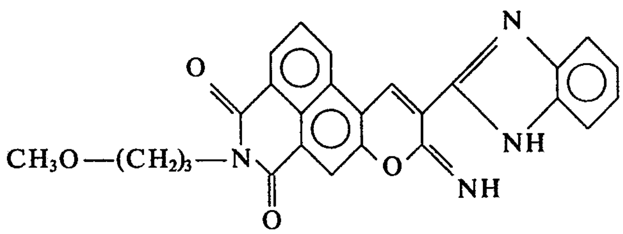

4. Coumarin compound of the formula

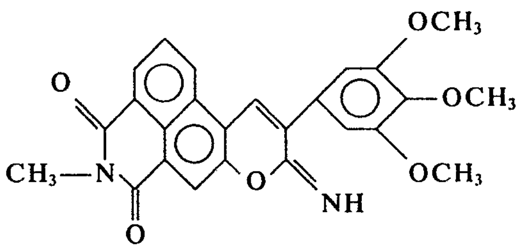

5. Coumarin compound of the formula

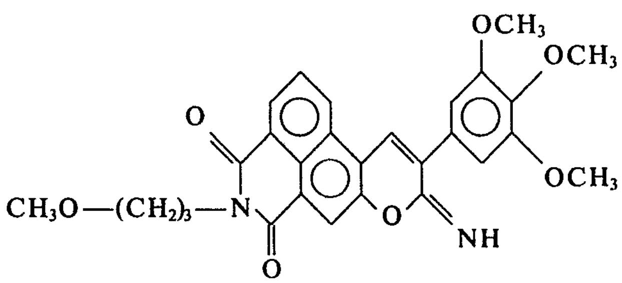

6. Coumarin compound of the formula

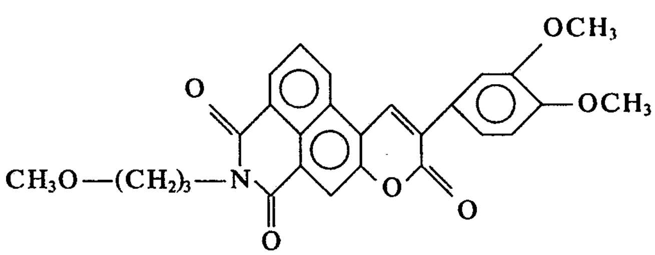

7. Coumarin compound of the formula

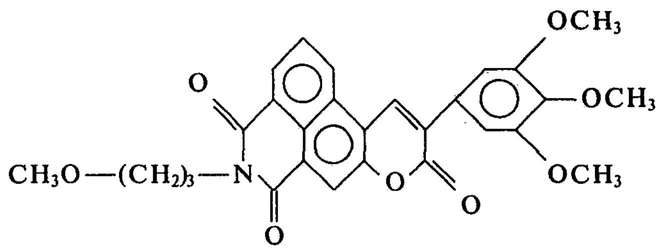

8. Process for preparing coumarin compounds of the general formula (1) as defined in claim 1, which comprises condensing a compound of the general formula (2)

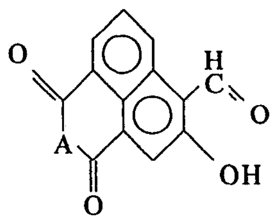

(2)

wherein A has the meanings mentioned in claim 1, with

(a) a compound of the general formula (3)

$NC-CH_2-Y$ (3)

wherein Y has the meanings mentioned in claim 1, in a manner which is in itself known to form an iminocoumarin compound of the general formula (4)

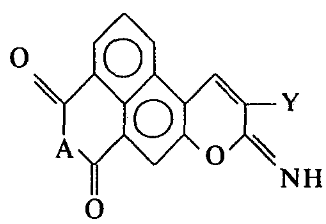

(4)

wherein A and Y have the meanings mentioned in claim 1, and thereafter acylating, treating with arylamines or hydrolyzing by methods which are in themselves known the resulting iminocoumarin compound of formula (4); or with

(b) a compound of the general formula (5)

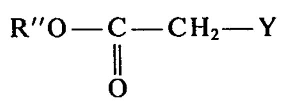

(5)

wherein Y has the meanings mentioned in claim 1 and R″ denotes an optionally substituted alkyl or aryl radical, in a manner which is in itself known to form a coumarin compound of the general formula (6)

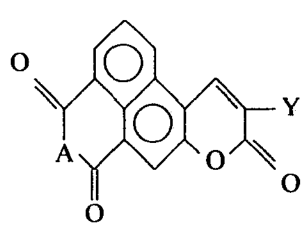

(6)

wherein A and Y have the meanings mentioned in claim 1, and, if desired, thereafter reacting the resulting coumarin compound of formula (6) by methods which are in themselves known with a cyanide salt and simultaneously or subsequently treating the addtion product which is thus formed as an intermediate with an oxidizing agent, thereby obtaining a coumarin compound of the general formula (7)

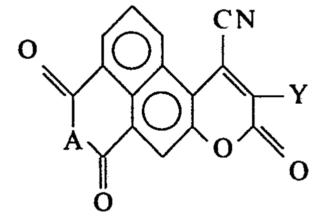

(7)

wherein A and Y have the meanings mentioned in claim 1.

9. Use of coumarin compounds of the general formula (1), as defined in claim 1, as dyestuffs.

10. Use according to claim 9, wherein the said dyestuffs are applied for dyeing or printing synthetic fiber materials, in particular those made of polyesters, optionally in admixture with natural fiber materials.

## Revendications

1. Composés coumariniques qui répondent à la formule générale 1:

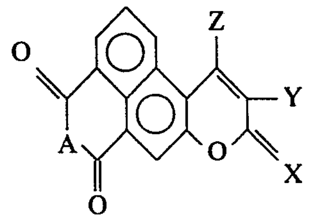

(1)

dans laquelle

A représente un atome d'oxygène ou un groupement >N—R dont le symbole R désigne:

- un atome d'hydrogène,
- un alkyle en $C_1$—$C_{18}$ éventuellement porteur d'atomes de fluor, de chlore ou de brome ou de radicaux alcoxy en $C_1$—$C_4$, dialkyl$_{\overline{C_1-C_4}}$amino, $-SO_2-N(alkyl_{C_1-C_4})_2$ ou cyano, ou
- un radical cycloaliphatique, aromatique ou hétérocyclique qui porte éventuellement des atomes de fluor, de chlore ou de brome ou des radicaux alkyles en $C_1$—$C_4$, alcoxy en $C_1$—$C_4$, hydroxy, cyano, sulfamoyle ou $-SO_1-N(alkyl_{C_1-C_4})_2$,

X représente un atome d'oxygène ou un radical =N—R' dont le symbole R' désigne:

- un atome d'hydrogène,
- un radical alkyl$_{\overline{C_1-C_{18}}}$carbonyle, alcoxy$_{\overline{C_1-C_4}}$carbonyle, alkyl$_{\overline{C_1-C_4}}$carbamoyle ou alkyl$_{\overline{C_1-C_4}}$sulfonyle, ou
- un radical aryle, arylcarbonyle, arylcarbamoyle ou arylsulfonyle, qui porte éventuellement des atomes de fluor, de chlore ou de brome, des radicaux alkyles ou alcoxy contenant chacun de 1 à 4 atomes de carbone ou des radicaux cyano,

Y représente un atome d'hydrogène, un radical cyano, un radical alcoxycarbonyle dont l'alkyle contient de 1 à 4 atomes de carbone, un radical aryle, arylsulfonyle ou aryloxycarbonyle, éventuellement porteur d'atomes de fluor, de chlore ou de brome, de radicaux alkyles ou de radicaux alcoxy contenant chacun de 1 à 4 atomes de carbone, de radicaux nitro, méthylène-dioxy, amino, monoalkyl$_{\overline{C_1-C_4}}$amino ou dialkyl$_{\overline{C_1-C_4}}$amino ou d'un radical benzoxazolyle, ou un radical hétérocyclique qui est éventuellement quaternisé et qui porte éventuellement des atomes de fluor, de chlore ou de brome, des radicaux alkyles ou des radicaux alcoxy contenant chacun de 1 à 4 atomes de carbone, ou des radicaux nitro, phényle, alkyl$_{\overline{C_1-C_4}}$phényles, alcoxy$_{\overline{C_1-C_4}}$phényles, cyano, méthylène-dioxy ou alcoxy$_{\overline{C_1-C_4}}$carbonyles,

Z représente un atome d'hydrogène, un radical alcoxy$_{\overline{C_1-C_4}}$carbonyle ou un radical cyano.

2. Composé coumarinique de formule:

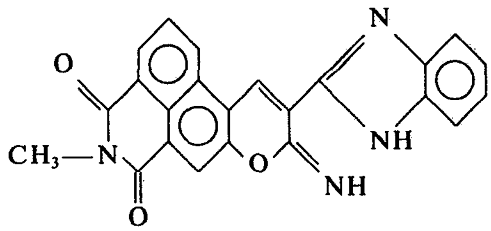

3. Composé coumarinique de formule:

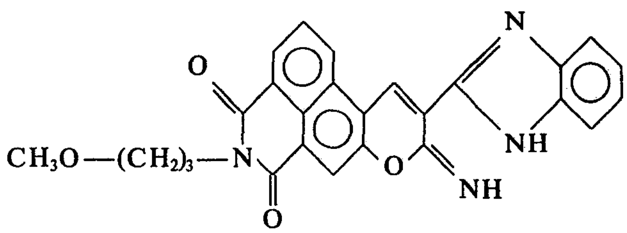

4. Composé coumarinique de formule:

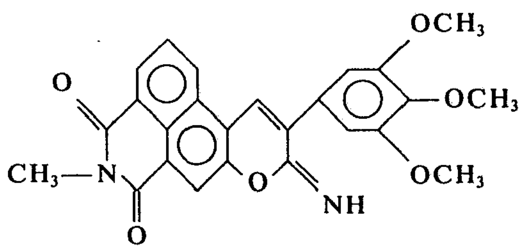

5. Composé coumarinique de formule:

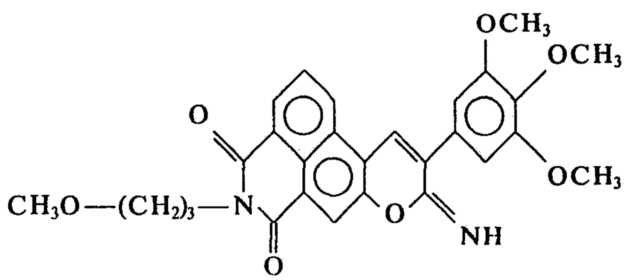

6. Composé coumarinique de formule:

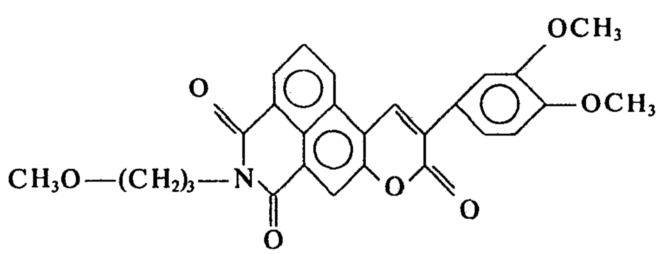

7. Composé coumarinique de formule:

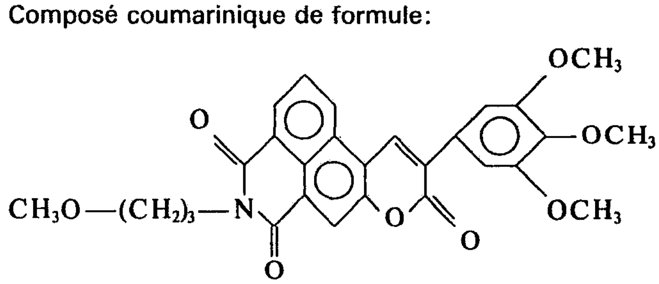

8. Procédé de préparation de composés coumariniques de formule générale 1 selon la revendication 1, procédé caractérisé en ce qu'on condense un composé répondant à la formule 2:

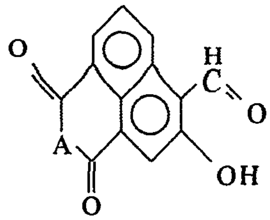

(2)

dans laquelle A a la signification donnée à la revendication 1,

– avec un composé répondant à la formule 3:

$$NC-CH_2-Y \qquad (3)$$

dans laquelle Y a la signification donnée à la revendication 1, de manière connue, et on acyle, on traite par des arylamines ou on saponifie le composé iminocoumarinique ainsi obtenu, qui répond à la formule générale 4:

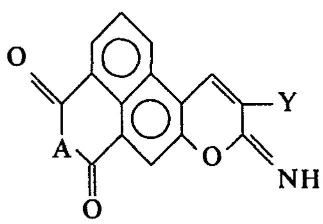

(4)

dans laquelle A et Y ont les significations données à la revendication 1, par des méthodes connues, ou

b) avec un composé répondant à la formule générale 5:

$$R''O-\underset{\underset{O}{\|}}{C}-CH_2-Y \qquad (5)$$

dans laquelle Y a la signification donnée à la revendication 1 et R" représente un radical alkyle ou aryle éventuellement substitué, de manière connue, éventuellement on fait réagir avec un sel de l'acide cyanhydrique, par des méthodes connues, le composé coumarinique ainsi obtenu, qui répond à la formule générale 6:

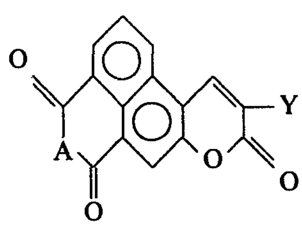

(6)

dans laquelle A et Y ont les significations données à la revendication 1, et on traite par un agent d'oxydation, en même temps ou ultérieurement, le composé d'addition qui s'est formé comme corps intermédiaire, ce traitement conduisant au composé de formule générale 7:

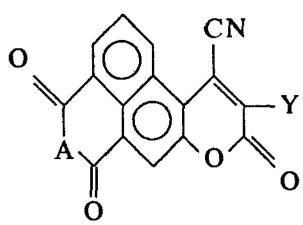

(7)

9. Application des composés de formule générale 1 qui ont été mentionnés à la revendication 1 en tant que colorants.

10. Application selon la revendication 9, caractérisée en ce qu'on utilise les colorants pour teindre ou imprimer des matières fibreuses synthétiques, plus spécialement en polyesters, éventuellement en mélange avec des matières fibreuses naturelles.